(19) Europäisches Patentamt / European Patent Office / Office européen des brevets

(11) EP 3 260 539 B1

(12) EUROPEAN PATENT SPECIFICATION

(45) Date of publication and mention of the grant of the patent: **06.10.2021 Bulletin 2021/40**

(21) Application number: **16752551.8**

(22) Date of filing: **18.02.2016**

(51) Int Cl.: *C12N 15/09* (2006.01) *A01K 67/027* (2006.01) *C12N 9/16* (2006.01)

(86) International application number: **PCT/JP2016/054735**

(87) International publication number: **WO 2016/133165 (25.08.2016 Gazette 2016/34)**

(54) **METHOD FOR TRANSFERRING CAS9 MRNA INTO MAMMALIAN FERTILIZED EGG BY ELECTROPORATION**

VERFAHREN ZUR ÜBERTRAGUNG VON CAS9-MRNA ZU EINEM BEFRUCHTETEN SÄUGEREI DURCH ELEKTROPORATION

PROCÉDÉ POUR TRANSFÉRER DE L’ARNM DE CAS9 DANS UN OVULE FÉCONDÉ DE MAMMIFÈRE PAR ÉLECTROPORATION

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2015 JP 2015031006**

(43) Date of publication of application: **27.12.2017 Bulletin 2017/52**

(73) Proprietors:
- **Tokushima University Tokushima 770-8501 (JP)**
- **Hashimoto, Masakazu Ibaraki-shi, Osaka 567-0046 (JP)**
- **Bex Co., Ltd. Tokyo 173-0004 (JP)**

(72) Inventors:
- **HASHIMOTO, Masakazu Ibaraki-shi Osaka 567-0046 (JP)**
- **TAKEMOTO, Tatsuya Tokushima-shi Tokushima 770-8501 (JP)**

(74) Representative: **Boult Wade Tennant LLP Salisbury Square House 8 Salisbury Square London EC4Y 8AP (GB)**

(56) References cited:
**WO-A1-2015/049897 WO-A1-2016/054032 JP-A- 2015 070 825**

- **TAKEHITO KANEKO ET AL: "Simple knockout by electroporation of engineered endonucleases into intact rat embryos", SCIENTIFIC REPORTS,, vol. 4, 1 October 2014 (2014-10-01), pages 1-5, XP002768158, DOI: 10.1038/SREP06382 [retrieved on 2014-10-01]**
- **HUI PENG ET AL: "Efficient Delivery of DNA and Morpholinos into Mouse Preimplantation Embryos by Electroporation", PLOS ONE, vol. 7, no. 8, 21 August 2012 (2012-08-21) , page e43748, XP055331643, DOI: 10.1371/journal.pone.0043748**
- **JOANNA B. GRABAREK ET AL: "Efficient delivery of dsRNA into zona-enclosed mouse oocytes and preimplantation embryos by electroporation", GENESIS: THE JOURNAL OF GENETICS AND DEVELOPMENT, vol. 32, no. 4, 1 April 2002 (2002-04-01), pages 269-276, XP055331639, US ISSN: 1526-954X, DOI: 10.1002/gene.10076**
- **HUI YANG ET AL: "One-Step Generation of Mice Carrying Reporter and Conditional Alleles by CRISPR/Cas-Mediated Genome Engineering (Includes Supplemental Informtion)", CELL, vol. 154, no. 6, 29 August 2013 (2013-08-29), page 1370, XP055462454, AMSTERDAM, NL ISSN: 0092-8674, DOI: 10.1016/j.cell.2013.08.022**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 260 539 B1

- KANEKO T ET AL.: 'Simple knockout by electroporation of engineered endonucleases into intact rat embryos.' SCI REP. vol. 4, 2014, page 6382, XP002768158
- HASHIMOTO M ET AL.: 'Electroporation enables the efficient mRNA delivery into the mouse zygotes and facilitates CRISPR/Cas9-based genome editing.' SCI REP. vol. 5, page 11315, XP055363790
- QIN W ET AL.: 'Efficient CRISPR/Cas9-Mediated Genome Editing in Mice by Zygote Electroporation of Nuclease.' GENETICS vol. 200, June 2015, pages 423 - 430, XP055363784

SEQUENCE LISTING

<110> Tokushima University BEX CO.,LTD. HASHIMOTO, MASAKAZU

<120> Method for introducing Cas9 mRNA into the mammalian fertilized eggs by electroporation

<130> 672694

<150> JP 2015-31006
<151> 2015-02-19

<160> 17

<170> PatentIn version 3.5

<210> 1
<211> 1368
<212> PRT
<213> Streptococcus pyogenes

<400> 1

```
Met Asp Lys Lys Tyr Ser Ile Gly Leu Asp Ile Gly Thr Asn Ser Val
1               5                   10                  15

Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160
```

```
Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
```

```
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655
```

```
Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp His Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910
```

```
Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys  Tyr Pro Lys Leu Glu  Ser Glu Phe
        995                 1000                1005

Val Tyr  Gly Asp Tyr Lys Val  Tyr Asp Val Arg Lys  Met Ile Ala
    1010                1015                1020

Lys Ser  Glu Gln Glu Ile Gly  Lys Ala Thr Ala Lys  Tyr Phe Phe
    1025                1030                1035

Tyr Ser  Asn Ile Met Asn Phe  Phe Lys Thr Glu Ile  Thr Leu Ala
    1040                1045                1050

Asn Gly  Glu Ile Arg Lys Arg  Pro Leu Ile Glu Thr  Asn Gly Glu
    1055                1060                1065

Thr Gly  Glu Ile Val Trp Asp  Lys Gly Arg Asp Phe  Ala Thr Val
    1070                1075                1080

Arg Lys  Val Leu Ser Met Pro  Gln Val Asn Ile Val  Lys Lys Thr
    1085                1090                1095

Glu Val  Gln Thr Gly Gly Phe  Ser Lys Glu Ser Ile  Leu Pro Lys
    1100                1105                1110

Arg Asn  Ser Asp Lys Leu Ile  Ala Arg Lys Lys Asp  Trp Asp Pro
    1115                1120                1125

Lys Lys  Tyr Gly Gly Phe Asp  Ser Pro Thr Val Ala  Tyr Ser Val
    1130                1135                1140

Leu Val  Val Ala Lys Val Glu  Lys Gly Lys Ser Lys  Lys Leu Lys
    1145                1150                1155
```

```
Ser Val  Lys Glu Leu Leu Gly  Ile Thr Ile Met Glu  Arg Ser Ser
    1160                 1165                 1170

Phe Glu  Lys Asn Pro Ile Asp  Phe Leu Glu Ala Lys  Gly Tyr Lys
    1175                 1180                 1185

Glu Val  Lys Lys Asp Leu Ile  Ile Lys Leu Pro Lys  Tyr Ser Leu
    1190                 1195                 1200

Phe Glu  Leu Glu Asn Gly Arg  Lys Arg Met Leu Ala  Ser Ala Gly
    1205                 1210                 1215

Glu Leu  Gln Lys Gly Asn Glu  Leu Ala Leu Pro Ser  Lys Tyr Val
    1220                 1225                 1230

Asn Phe  Leu Tyr Leu Ala Ser  His Tyr Glu Lys Leu  Lys Gly Ser
    1235                 1240                 1245

Pro Glu  Asp Asn Glu Gln Lys  Gln Leu Phe Val Glu  Gln His Lys
    1250                 1255                 1260

His Tyr  Leu Asp Glu Ile Ile  Glu Gln Ile Ser Glu  Phe Ser Lys
    1265                 1270                 1275

Arg Val  Ile Leu Ala Asp Ala  Asn Leu Asp Lys Val  Leu Ser Ala
    1280                 1285                 1290

Tyr Asn  Lys His Arg Asp Lys  Pro Ile Arg Glu Gln  Ala Glu Asn
    1295                 1300                 1305

Ile Ile  His Leu Phe Thr Leu  Thr Asn Leu Gly Ala  Pro Ala Ala
    1310                 1315                 1320

Phe Lys  Tyr Phe Asp Thr Thr  Ile Asp Arg Lys Arg  Tyr Thr Ser
    1325                 1330                 1335

Thr Lys  Glu Val Leu Asp Ala  Thr Leu Ile His Gln  Ser Ile Thr
    1340                 1345                 1350

Gly Leu  Tyr Glu Thr Arg Ile  Asp Leu Ser Gln Leu  Gly Gly Asp
    1355                 1360                 1365
```

<210> 2
<211> 1082
<212> PRT
<213> Neisseria meningitidis

<400> 2

```
Met Ala Ala Phe Lys Pro Asn Ser Ile Asn Tyr Ile Leu Gly Leu Asp
1               5                   10                  15

Ile Gly Ile Ala Ser Val Gly Trp Ala Met Val Glu Ile Asp Glu Glu
            20                  25                  30

Glu Asn Pro Ile Arg Leu Ile Asp Leu Gly Val Arg Val Phe Glu Arg
        35                  40                  45

Ala Glu Val Pro Lys Thr Gly Asp Ser Leu Ala Met Ala Arg Arg Leu
    50                  55                  60

Ala Arg Ser Val Arg Arg Leu Thr Arg Arg Arg Ala His Arg Leu Leu
65                  70                  75                  80

Arg Thr Arg Arg Leu Leu Lys Arg Glu Gly Val Leu Gln Ala Ala Asn
                85                  90                  95

Phe Asp Glu Asn Gly Leu Ile Lys Ser Leu Pro Asn Thr Pro Trp Gln
            100                 105                 110

Leu Arg Ala Ala Ala Leu Asp Arg Lys Leu Thr Pro Leu Glu Trp Ser
        115                 120                 125

Ala Val Leu Leu His Leu Ile Lys His Arg Gly Tyr Leu Ser Gln Arg
    130                 135                 140

Lys Asn Glu Gly Glu Thr Ala Asp Lys Glu Leu Gly Ala Leu Leu Lys
145                 150                 155                 160

Gly Val Ala Gly Asn Ala His Ala Leu Gln Thr Gly Asp Phe Arg Thr
                165                 170                 175

Pro Ala Glu Leu Ala Leu Asn Lys Phe Glu Lys Glu Ser Gly His Ile
            180                 185                 190

Arg Asn Gln Arg Ser Asp Tyr Ser His Thr Phe Ser Arg Lys Asp Leu
        195                 200                 205

Gln Ala Glu Leu Ile Leu Leu Phe Glu Lys Gln Lys Glu Phe Gly Asn
    210                 215                 220

Pro His Val Ser Gly Gly Leu Lys Glu Gly Ile Glu Thr Leu Leu Met
225                 230                 235                 240

Thr Gln Arg Pro Ala Leu Ser Gly Asp Ala Val Gln Lys Met Leu Gly
```

```
                245                 250                 255

His Cys Thr Phe Glu Pro Ala Glu Pro Lys Ala Ala Lys Asn Thr Tyr
            260                 265                 270

Thr Ala Glu Arg Phe Ile Trp Leu Thr Lys Leu Asn Asn Leu Arg Ile
        275                 280                 285

Leu Glu Gln Gly Ser Glu Arg Pro Leu Thr Asp Thr Glu Arg Ala Thr
    290                 295                 300

Leu Met Asp Glu Pro Tyr Arg Lys Ser Lys Leu Thr Tyr Ala Gln Ala
305                 310                 315                 320

Arg Lys Leu Leu Gly Leu Glu Asp Thr Ala Phe Phe Lys Gly Leu Arg
                325                 330                 335

Tyr Gly Lys Asp Asn Ala Glu Ala Ser Thr Leu Met Glu Met Lys Ala
            340                 345                 350

Tyr His Ala Ile Ser Arg Ala Leu Glu Lys Glu Gly Leu Lys Asp Lys
        355                 360                 365

Lys Ser Pro Leu Asn Leu Ser Pro Glu Leu Gln Asp Glu Ile Gly Thr
    370                 375                 380

Ala Phe Ser Leu Phe Lys Thr Asp Glu Asp Ile Thr Gly Arg Leu Lys
385                 390                 395                 400

Asp Arg Ile Gln Pro Glu Ile Leu Glu Ala Leu Leu Lys His Ile Ser
                405                 410                 415

Phe Asp Lys Phe Val Gln Ile Ser Leu Lys Ala Leu Arg Arg Ile Val
            420                 425                 430

Pro Leu Met Glu Gln Gly Lys Arg Tyr Asp Glu Ala Cys Ala Glu Ile
        435                 440                 445

Tyr Gly Asp His Tyr Gly Lys Lys Asn Thr Glu Glu Lys Ile Tyr Leu
    450                 455                 460

Pro Pro Ile Pro Ala Asp Glu Ile Arg Asn Pro Val Val Leu Arg Ala
465                 470                 475                 480

Leu Ser Gln Ala Arg Lys Val Ile Asn Gly Val Val Arg Arg Tyr Gly
                485                 490                 495
```

```
Ser Pro Ala Arg Ile His Ile Glu Thr Ala Arg Glu Val Gly Lys Ser
            500                 505                 510

Phe Lys Asp Arg Lys Glu Ile Glu Lys Arg Gln Glu Glu Asn Arg Lys
        515                 520                 525

Asp Arg Glu Lys Ala Ala Ala Lys Phe Arg Glu Tyr Phe Pro Asn Phe
    530                 535                 540

Val Gly Glu Pro Lys Ser Lys Asp Ile Leu Lys Leu Arg Leu Tyr Glu
545                 550                 555                 560

Gln Gln His Gly Lys Cys Leu Tyr Ser Gly Lys Glu Ile Asn Leu Gly
                565                 570                 575

Arg Leu Asn Glu Lys Gly Tyr Val Glu Ile Asp His Ala Leu Pro Phe
            580                 585                 590

Ser Arg Thr Trp Asp Asp Ser Phe Asn Asn Lys Val Leu Val Leu Gly
        595                 600                 605

Ser Glu Asn Gln Asn Lys Gly Asn Gln Thr Pro Tyr Glu Tyr Phe Asn
    610                 615                 620

Gly Lys Asp Asn Ser Arg Glu Trp Gln Glu Phe Lys Ala Arg Val Glu
625                 630                 635                 640

Thr Ser Arg Phe Pro Arg Ser Lys Lys Gln Arg Ile Leu Leu Gln Lys
                645                 650                 655

Phe Asp Glu Asp Gly Phe Lys Glu Arg Asn Leu Asn Asp Thr Arg Tyr
            660                 665                 670

Val Asn Arg Phe Leu Cys Gln Phe Val Ala Asp Arg Met Arg Leu Thr
        675                 680                 685

Gly Lys Gly Lys Lys Arg Val Phe Ala Ser Asn Gly Gln Ile Thr Asn
    690                 695                 700

Leu Leu Arg Gly Phe Trp Gly Leu Arg Lys Val Arg Ala Glu Asn Asp
705                 710                 715                 720

Arg His His Ala Leu Asp Ala Val Val Val Ala Cys Ser Thr Val Ala
                725                 730                 735

Met Gln Gln Lys Ile Thr Arg Phe Val Arg Tyr Lys Glu Met Asn Ala
            740                 745                 750
```

```
Phe Asp Gly Lys Thr Ile Asp Lys Glu Thr Gly Glu Val Leu His Gln
        755                 760                 765

Lys Thr His Phe Pro Gln Pro Trp Glu Phe Phe Ala Gln Glu Val Met
    770                 775                 780

Ile Arg Val Phe Gly Lys Pro Asp Gly Lys Pro Glu Phe Glu Glu Ala
785                 790                 795                 800

Asp Thr Leu Glu Lys Leu Arg Thr Leu Leu Ala Glu Lys Leu Ser Ser
                805                 810                 815

Arg Pro Glu Ala Val His Glu Tyr Val Thr Pro Leu Phe Val Ser Arg
            820                 825                 830

Ala Pro Asn Arg Lys Met Ser Gly Gln Gly His Met Glu Thr Val Lys
        835                 840                 845

Ser Ala Lys Arg Leu Asp Glu Gly Val Ser Val Leu Arg Val Pro Leu
    850                 855                 860

Thr Gln Leu Lys Leu Lys Asp Leu Glu Lys Met Val Asn Arg Glu Arg
865                 870                 875                 880

Glu Pro Lys Leu Tyr Glu Ala Leu Lys Ala Arg Leu Glu Ala His Lys
                885                 890                 895

Asp Asp Pro Ala Lys Ala Phe Ala Glu Pro Phe Tyr Lys Tyr Asp Lys
            900                 905                 910

Ala Gly Asn Arg Thr Gln Gln Val Lys Ala Val Arg Val Glu Gln Val
        915                 920                 925

Gln Lys Thr Gly Val Trp Val Arg Asn His Asn Gly Ile Ala Asp Asn
    930                 935                 940

Ala Thr Met Val Arg Val Asp Val Phe Glu Lys Gly Asp Lys Tyr Tyr
945                 950                 955                 960

Leu Val Pro Ile Tyr Ser Trp Gln Val Ala Lys Gly Ile Leu Pro Asp
                965                 970                 975

Arg Ala Val Val Gln Gly Lys Asp Glu Glu Asp Trp Gln Leu Ile Asp
            980                 985                 990

Asp Ser Phe Asn Phe Lys Phe Ser  Leu His Pro Asn Asp  Leu Val Glu
        995                 1000                1005
```

```
Val Ile  Thr Lys Lys Ala Arg  Met Phe Gly Tyr Phe  Ala Ser Cys
    1010                 1015                 1020

His Arg  Gly Thr Gly Asn Ile  Asn Ile Arg Ile His  Asp Leu Asp
    1025                 1030                 1035

His Lys  Ile Gly Lys Asn Gly  Ile Leu Glu Gly Ile  Gly Val Lys
    1040                 1045                 1050

Thr Ala  Leu Ser Phe Gln Lys  Tyr Gln Ile Asp Glu  Leu Gly Lys
    1055                 1060                 1065

Glu Ile  Arg Pro Cys Arg Leu  Lys Lys Arg Pro Pro  Val Arg
    1070                 1075                 1080


<210>  3
<211>  1388
<212>  PRT
<213>  Streptococcus thermophilus

<400>  3

Met Thr Lys Pro Tyr Ser Ile Gly Leu Asp Ile Gly Thr Asn Ser Val
1               5                   10                  15

Gly Trp Ala Val Thr Thr Asp Asn Tyr Lys Val Pro Ser Lys Lys Met
            20                  25                  30

Lys Val Leu Gly Asn Thr Ser Lys Lys Tyr Ile Lys Lys Asn Leu Leu
        35                  40                  45

Gly Val Leu Leu Phe Asp Ser Gly Ile Thr Ala Glu Gly Arg Arg Leu
    50                  55                  60

Lys Arg Thr Ala Arg Arg Arg Tyr Thr Arg Arg Arg Asn Arg Ile Leu
65                  70                  75                  80

Tyr Leu Gln Glu Ile Phe Ser Thr Glu Met Ala Thr Leu Asp Asp Ala
                85                  90                  95

Phe Phe Gln Arg Leu Asp Asp Ser Phe Leu Val Pro Asp Asp Lys Arg
            100                 105                 110

Asp Ser Lys Tyr Pro Ile Phe Gly Asn Leu Val Glu Glu Lys Ala Tyr
        115                 120                 125

His Asp Glu Phe Pro Thr Ile Tyr His Leu Arg Lys Tyr Leu Ala Asp
    130                 135                 140
```

```
Ser Thr Lys Lys Ala Asp Leu Arg Leu Val Tyr Leu Ala Leu Ala His
145                 150                 155                 160

Met Ile Lys Tyr Arg Gly His Phe Leu Ile Glu Gly Glu Phe Asn Ser
                165                 170                 175

Lys Asn Asn Asp Ile Gln Lys Asn Phe Gln Asp Phe Leu Asp Thr Tyr
            180                 185                 190

Asn Ala Ile Phe Glu Ser Asp Leu Ser Leu Glu Asn Ser Lys Gln Leu
        195                 200                 205

Glu Glu Ile Val Lys Asp Lys Ile Ser Lys Leu Glu Lys Lys Asp Arg
    210                 215                 220

Ile Leu Lys Leu Phe Pro Gly Glu Lys Asn Ser Gly Ile Phe Ser Glu
225                 230                 235                 240

Phe Leu Lys Leu Ile Val Gly Asn Gln Ala Asp Phe Arg Lys Cys Phe
                245                 250                 255

Asn Leu Asp Glu Lys Ala Ser Leu His Phe Ser Lys Glu Ser Tyr Asp
            260                 265                 270

Glu Asp Leu Glu Thr Leu Leu Gly Tyr Ile Gly Asp Asp Tyr Ser Asp
        275                 280                 285

Val Phe Leu Lys Ala Lys Lys Leu Tyr Asp Ala Ile Leu Leu Ser Gly
    290                 295                 300

Phe Leu Thr Val Thr Asp Asn Glu Thr Glu Ala Pro Leu Ser Ser Ala
305                 310                 315                 320

Met Ile Lys Arg Tyr Asn Glu His Lys Glu Asp Leu Ala Leu Leu Lys
                325                 330                 335

Glu Tyr Ile Arg Asn Ile Ser Leu Lys Thr Tyr Asn Glu Val Phe Lys
            340                 345                 350

Asp Asp Thr Lys Asn Gly Tyr Ala Gly Tyr Ile Asp Gly Lys Thr Asn
        355                 360                 365

Gln Glu Asp Phe Tyr Val Tyr Leu Lys Lys Leu Leu Ala Glu Phe Glu
    370                 375                 380

Gly Ala Asp Tyr Phe Leu Glu Lys Ile Asp Arg Glu Asp Phe Leu Arg
```

```
385                 390                 395                 400

Lys Gln Arg Thr Phe Asp Asn Gly Ser Ile Pro Tyr Gln Ile His Leu
                405                 410                 415

Gln Glu Met Arg Ala Ile Leu Asp Lys Gln Ala Lys Phe Tyr Pro Phe
            420                 425                 430

Leu Ala Lys Asn Lys Glu Arg Ile Glu Lys Ile Leu Thr Phe Arg Ile
        435                 440                 445

Pro Tyr Tyr Val Gly Pro Leu Ala Arg Gly Asn Ser Asp Phe Ala Trp
    450                 455                 460

Ser Ile Arg Lys Arg Asn Glu Lys Ile Thr Pro Trp Asn Phe Glu Asp
465                 470                 475                 480

Val Ile Asp Lys Glu Ser Ser Ala Glu Ala Phe Ile Asn Arg Met Thr
                485                 490                 495

Ser Phe Asp Leu Tyr Leu Pro Glu Glu Lys Val Leu Pro Lys His Ser
            500                 505                 510

Leu Leu Tyr Glu Thr Phe Asn Val Tyr Asn Glu Leu Thr Lys Val Arg
        515                 520                 525

Phe Ile Ala Glu Ser Met Arg Asp Tyr Gln Phe Leu Asp Ser Lys Gln
    530                 535                 540

Lys Lys Asp Ile Val Arg Leu Tyr Phe Lys Asp Lys Arg Lys Val Thr
545                 550                 555                 560

Asp Lys Asp Ile Ile Glu Tyr Leu His Ala Ile Tyr Gly Tyr Asp Gly
                565                 570                 575

Ile Glu Leu Lys Gly Ile Glu Lys Gln Phe Asn Ser Ser Leu Ser Thr
            580                 585                 590

Tyr His Asp Leu Leu Asn Ile Ile Asn Asp Lys Glu Phe Leu Asp Asp
        595                 600                 605

Ser Ser Asn Glu Ala Ile Ile Glu Glu Ile Ile His Thr Leu Thr Ile
    610                 615                 620

Phe Glu Asp Arg Glu Met Ile Lys Gln Arg Leu Ser Lys Phe Glu Asn
625                 630                 635                 640
```

```
Ile Phe Asp Lys Ser Val Leu Lys Lys Leu Ser Arg Arg His Tyr Thr
                645                 650                 655

Gly Trp Gly Lys Leu Ser Ala Lys Leu Ile Asn Gly Ile Arg Asp Glu
            660                 665                 670

Lys Ser Gly Asn Thr Ile Leu Asp Tyr Leu Ile Asp Asp Gly Ile Ser
        675                 680                 685

Asn Arg Asn Phe Met Gln Leu Ile His Asp Asp Ala Leu Ser Phe Lys
    690                 695                 700

Lys Lys Ile Gln Lys Ala Gln Ile Ile Gly Asp Glu Asp Lys Gly Asn
705                 710                 715                 720

Ile Lys Glu Val Val Lys Ser Leu Pro Gly Ser Pro Ala Ile Lys Lys
                725                 730                 735

Gly Ile Leu Gln Ser Ile Lys Ile Val Asp Glu Leu Val Lys Val Met
            740                 745                 750

Gly Gly Arg Lys Pro Glu Ser Ile Val Val Glu Met Ala Arg Glu Asn
        755                 760                 765

Gln Tyr Thr Asn Gln Gly Lys Ser Asn Ser Gln Gln Arg Leu Lys Arg
    770                 775                 780

Leu Glu Lys Ser Leu Lys Glu Leu Gly Ser Lys Ile Leu Lys Glu Asn
785                 790                 795                 800

Ile Pro Ala Lys Leu Ser Lys Ile Asp Asn Asn Ala Leu Gln Asn Asp
                805                 810                 815

Arg Leu Tyr Leu Tyr Tyr Leu Gln Asn Gly Lys Asp Met Tyr Thr Gly
            820                 825                 830

Asp Asp Leu Asp Ile Asp Arg Leu Ser Asn Tyr Asp Ile Asp His Ile
        835                 840                 845

Ile Pro Gln Ala Phe Leu Lys Asp Asn Ser Ile Asp Asn Lys Val Leu
    850                 855                 860

Val Ser Ser Ala Ser Asn Arg Gly Lys Ser Asp Asp Val Pro Ser Leu
865                 870                 875                 880

Glu Val Val Lys Lys Arg Lys Thr Phe Trp Tyr Gln Leu Leu Lys Ser
                885                 890                 895
```

```
Lys Leu Ile Ser Gln Arg Lys Phe Asp Asn Leu Thr Lys Ala Glu Arg
            900                 905                 910

Gly Gly Leu Ser Pro Glu Asp Lys Ala Gly Phe Ile Gln Arg Gln Leu
        915                 920                 925

Val Glu Thr Arg Gln Ile Thr Lys His Val Ala Arg Leu Leu Asp Glu
    930                 935                 940

Lys Phe Asn Asn Lys Lys Asp Glu Asn Asn Arg Ala Val Arg Thr Val
945                 950                 955                 960

Lys Ile Ile Thr Leu Lys Ser Thr Leu Val Ser Gln Phe Arg Lys Asp
                965                 970                 975

Phe Glu Leu Tyr Lys Val Arg Glu Ile Asn Asp Phe His His Ala His
            980                 985                 990

Asp Ala Tyr Leu Asn Ala Val Val  Ala Ser Ala Leu Leu  Lys Lys Tyr
        995                 1000                1005

Pro Lys  Leu Glu Pro Glu Phe  Val Tyr Gly Asp Tyr  Pro Lys Tyr
    1010                1015                1020

Asn Ser  Phe Arg Glu Arg Lys  Ser Ala Thr Glu Lys  Val Tyr Phe
    1025                1030                1035

Tyr Ser  Asn Ile Met Asn Ile  Phe Lys Lys Ser Ile  Ser Leu Ala
    1040                1045                1050

Asp Gly  Arg Val Ile Glu Arg  Pro Leu Ile Glu Val  Asn Glu Glu
    1055                1060                1065

Thr Gly  Glu Ser Val Trp Asn  Lys Glu Ser Asp Leu  Ala Thr Val
    1070                1075                1080

Arg Arg  Val Leu Ser Tyr Pro  Gln Val Asn Val Val  Lys Lys Val
    1085                1090                1095

Glu Glu  Gln Asn His Gly Leu  Asp Arg Gly Lys Pro  Lys Gly Leu
    1100                1105                1110

Phe Asn  Ala Asn Leu Ser Ser  Lys Pro Lys Pro Asn  Ser Asn Glu
    1115                1120                1125

Asn Leu  Val Gly Ala Lys Glu  Tyr Leu Asp Pro Lys  Lys Tyr Gly
    1130                1135                1140
```

```
Gly Tyr  Ala Gly Ile Ser Asn  Ser Phe Thr Val Leu  Val Lys Gly
    1145                 1150                 1155

Thr Ile  Glu Lys Gly Ala Lys  Lys Lys Ile Thr Asn  Val Leu Glu
    1160                 1165                 1170

Phe Gln  Gly Ile Ser Ile Leu  Asp Arg Ile Asn Tyr  Arg Lys Asp
    1175                 1180                 1185

Lys Leu  Asn Phe Leu Leu Glu  Lys Gly Tyr Lys Asp  Ile Glu Leu
    1190                 1195                 1200

Ile Ile  Glu Leu Pro Lys Tyr  Ser Leu Phe Glu Leu  Ser Asp Gly
    1205                 1210                 1215

Ser Arg  Arg Met Leu Ala Ser  Ile Leu Ser Thr Asn  Asn Lys Arg
    1220                 1225                 1230

Gly Glu  Ile His Lys Gly Asn  Gln Ile Phe Leu Ser  Gln Lys Phe
    1235                 1240                 1245

Val Lys  Leu Leu Tyr His Ala  Lys Arg Ile Ser Asn  Thr Ile Asn
    1250                 1255                 1260

Glu Asn  His Arg Lys Tyr Val  Glu Asn His Lys Lys  Glu Phe Glu
    1265                 1270                 1275

Glu Leu  Phe Tyr Tyr Ile Leu  Glu Phe Asn Glu Asn  Tyr Val Gly
    1280                 1285                 1290

Ala Lys  Lys Asn Gly Lys Leu  Leu Asn Ser Ala Phe  Gln Ser Trp
    1295                 1300                 1305

Gln Asn  His Ser Ile Asp Glu  Leu Cys Ser Ser Phe  Ile Gly Pro
    1310                 1315                 1320

Thr Gly  Ser Glu Arg Lys Gly  Leu Phe Glu Leu Thr  Ser Arg Gly
    1325                 1330                 1335

Ser Ala  Ala Asp Phe Glu Phe  Leu Gly Val Lys Ile  Pro Arg Tyr
    1340                 1345                 1350

Arg Asp  Tyr Thr Pro Ser Ser  Leu Leu Lys Asp Ala  Thr Leu Ile
    1355                 1360                 1365

His Gln  Ser Val Thr Gly Leu  Tyr Glu Thr Arg Ile  Asp Leu Ala
```

1370 1375 1380

Lys Leu Gly Glu Gly
1385

<210> 4
<211> 1395
<212> PRT
<213> Treponema denticola

<400> 4

```
Met Lys Lys Glu Ile Lys Asp Tyr Phe Leu Gly Leu Asp Val Gly Thr
1               5                   10                  15

Gly Ser Val Gly Trp Ala Val Thr Asp Thr Asp Tyr Lys Leu Leu Lys
            20                  25                  30

Ala Asn Arg Lys Asp Leu Trp Gly Met Arg Cys Phe Glu Thr Ala Glu
        35                  40                  45

Thr Ala Glu Val Arg Arg Leu His Arg Gly Ala Arg Arg Arg Ile Glu
    50                  55                  60

Arg Arg Lys Lys Arg Ile Lys Leu Leu Gln Glu Leu Phe Ser Gln Glu
65                  70                  75                  80

Ile Ala Lys Thr Asp Glu Gly Phe Phe Gln Arg Met Lys Glu Ser Pro
                85                  90                  95

Phe Tyr Ala Glu Asp Lys Thr Ile Leu Gln Glu Asn Thr Leu Phe Asn
            100                 105                 110

Asp Lys Asp Phe Ala Asp Lys Thr Tyr His Lys Ala Tyr Pro Thr Ile
        115                 120                 125

Asn His Leu Ile Lys Ala Trp Ile Glu Asn Lys Val Lys Pro Asp Pro
    130                 135                 140

Arg Leu Leu Tyr Leu Ala Cys His Asn Ile Ile Lys Lys Arg Gly His
145                 150                 155                 160

Phe Leu Phe Glu Gly Asp Phe Asp Ser Glu Asn Gln Phe Asp Thr Ser
                165                 170                 175

Ile Gln Ala Leu Phe Glu Tyr Leu Arg Glu Asp Met Glu Val Asp Ile
            180                 185                 190

Asp Ala Asp Ser Gln Lys Val Lys Glu Ile Leu Lys Asp Ser Ser Leu
```

```
            195                 200                 205

Lys Asn Ser Glu Lys Gln Ser Arg Leu Asn Lys Ile Leu Gly Leu Lys
    210                 215                 220

Pro Ser Asp Lys Gln Lys Lys Ala Ile Thr Asn Leu Ile Ser Gly Asn
225                 230                 235                 240

Lys Ile Asn Phe Ala Asp Leu Tyr Asp Asn Pro Asp Leu Lys Asp Ala
                245                 250                 255

Glu Lys Asn Ser Ile Ser Phe Ser Lys Asp Asp Phe Asp Ala Leu Ser
            260                 265                 270

Asp Asp Leu Ala Ser Ile Leu Gly Asp Ser Phe Glu Leu Leu Leu Lys
        275                 280                 285

Ala Lys Ala Val Tyr Asn Cys Ser Val Leu Ser Lys Val Ile Gly Asp
    290                 295                 300

Glu Gln Tyr Leu Ser Phe Ala Lys Val Lys Ile Tyr Glu Lys His Lys
305                 310                 315                 320

Thr Asp Leu Thr Lys Leu Lys Asn Val Ile Lys Lys His Phe Pro Lys
                325                 330                 335

Asp Tyr Lys Lys Val Phe Gly Tyr Asn Lys Asn Glu Lys Asn Asn Asn
            340                 345                 350

Asn Tyr Ser Gly Tyr Val Gly Val Cys Lys Thr Lys Ser Lys Lys Leu
        355                 360                 365

Ile Ile Asn Asn Ser Val Asn Gln Glu Asp Phe Tyr Lys Phe Leu Lys
    370                 375                 380

Thr Ile Leu Ser Ala Lys Ser Glu Ile Lys Glu Val Asn Asp Ile Leu
385                 390                 395                 400

Thr Glu Ile Glu Thr Gly Thr Phe Leu Pro Lys Gln Ile Ser Lys Ser
                405                 410                 415

Asn Ala Glu Ile Pro Tyr Gln Leu Arg Lys Met Glu Leu Glu Lys Ile
            420                 425                 430

Leu Ser Asn Ala Glu Lys His Phe Ser Phe Leu Lys Gln Lys Asp Glu
        435                 440                 445
```

```
Lys Gly Leu Ser His Ser Glu Lys Ile Ile Met Leu Leu Thr Phe Lys
    450                 455                 460

Ile Pro Tyr Tyr Ile Gly Pro Ile Asn Asp Asn His Lys Lys Phe Phe
465                 470                 475                 480

Pro Asp Arg Cys Trp Val Val Lys Lys Glu Lys Ser Pro Ser Gly Lys
                485                 490                 495

Thr Thr Pro Trp Asn Phe Phe Asp His Ile Asp Lys Glu Lys Thr Ala
            500                 505                 510

Glu Ala Phe Ile Thr Ser Arg Thr Asn Phe Cys Thr Tyr Leu Val Gly
        515                 520                 525

Glu Ser Val Leu Pro Lys Ser Ser Leu Leu Tyr Ser Glu Tyr Thr Val
    530                 535                 540

Leu Asn Glu Ile Asn Asn Leu Gln Ile Ile Ile Asp Gly Lys Asn Ile
545                 550                 555                 560

Cys Asp Ile Lys Leu Lys Gln Lys Ile Tyr Glu Asp Leu Phe Lys Lys
                565                 570                 575

Tyr Lys Lys Ile Thr Gln Lys Gln Ile Ser Thr Phe Ile Lys His Glu
            580                 585                 590

Gly Ile Cys Asn Lys Thr Asp Glu Val Ile Ile Leu Gly Ile Asp Lys
        595                 600                 605

Glu Cys Thr Ser Ser Leu Lys Ser Tyr Ile Glu Leu Lys Asn Ile Phe
    610                 615                 620

Gly Lys Gln Val Asp Glu Ile Ser Thr Lys Asn Met Leu Glu Glu Ile
625                 630                 635                 640

Ile Arg Trp Ala Thr Ile Tyr Asp Glu Gly Glu Gly Lys Thr Ile Leu
                645                 650                 655

Lys Thr Lys Ile Lys Ala Glu Tyr Gly Lys Tyr Cys Ser Asp Glu Gln
            660                 665                 670

Ile Lys Lys Ile Leu Asn Leu Lys Phe Ser Gly Trp Gly Arg Leu Ser
        675                 680                 685

Arg Lys Phe Leu Glu Thr Val Thr Ser Glu Met Pro Gly Phe Ser Glu
    690                 695                 700
```

```
Pro Val Asn Ile Ile Thr Ala Met Arg Glu Thr Gln Asn Asn Leu Met
705                 710                 715                 720

Glu Leu Leu Ser Ser Glu Phe Thr Phe Thr Glu Asn Ile Lys Lys Ile
                725                 730                 735

Asn Ser Gly Phe Glu Asp Ala Glu Lys Gln Phe Ser Tyr Asp Gly Leu
            740                 745                 750

Val Lys Pro Leu Phe Leu Ser Pro Ser Val Lys Lys Met Leu Trp Gln
        755                 760                 765

Thr Leu Lys Leu Val Lys Glu Ile Ser His Ile Thr Gln Ala Pro Pro
    770                 775                 780

Lys Lys Ile Phe Ile Glu Met Ala Lys Gly Ala Glu Leu Glu Pro Ala
785                 790                 795                 800

Arg Thr Lys Thr Arg Leu Lys Ile Leu Gln Asp Leu Tyr Asn Asn Cys
                805                 810                 815

Lys Asn Asp Ala Asp Ala Phe Ser Ser Glu Ile Lys Asp Leu Ser Gly
            820                 825                 830

Lys Ile Glu Asn Glu Asp Asn Leu Arg Leu Arg Ser Asp Lys Leu Tyr
        835                 840                 845

Leu Tyr Tyr Thr Gln Leu Gly Lys Cys Met Tyr Cys Gly Lys Pro Ile
    850                 855                 860

Glu Ile Gly His Val Phe Asp Thr Ser Asn Tyr Asp Ile Asp His Ile
865                 870                 875                 880

Tyr Pro Gln Ser Lys Ile Lys Asp Asp Ser Ile Ser Asn Arg Val Leu
                885                 890                 895

Val Cys Ser Ser Cys Asn Lys Asn Lys Glu Asp Lys Tyr Pro Leu Lys
            900                 905                 910

Ser Glu Ile Gln Ser Lys Gln Arg Gly Phe Trp Asn Phe Leu Gln Arg
        915                 920                 925

Asn Asn Phe Ile Ser Leu Glu Lys Leu Asn Arg Leu Thr Arg Ala Thr
    930                 935                 940

Pro Ile Ser Asp Asp Glu Thr Ala Lys Phe Ile Ala Arg Gln Leu Val
945                 950                 955                 960
```

```
Glu Thr Arg Gln Ala Thr Lys Val Ala Ala Lys Val Leu Glu Lys Met
                965                 970                 975

Phe Pro Glu Thr Lys Ile Val Tyr Ser Lys Ala Glu Thr Val Ser Met
            980                 985                 990

Phe Arg Asn Lys Phe Asp Ile Val  Lys Cys Arg Glu Ile  Asn Asp Phe
        995                 1000                 1005

His His  Ala His Asp Ala Tyr  Leu Asn Ile Val Val  Gly Asn Val
    1010                 1015                 1020

Tyr Asn  Thr Lys Phe Thr Asn  Asn Pro Trp Asn Phe  Ile Lys Glu
    1025                 1030                 1035

Lys Arg  Asp Asn Pro Lys Ile  Ala Asp Thr Tyr Asn  Tyr Tyr Lys
    1040                 1045                 1050

Val Phe  Asp Tyr Asp Val Lys  Arg Asn Asn Ile Thr  Ala Trp Glu
    1055                 1060                 1065

Lys Gly  Lys Thr Ile Ile Thr  Val Lys Asp Met Leu  Lys Arg Asn
    1070                 1075                 1080

Thr Pro  Ile Tyr Thr Arg Gln  Ala Ala Cys Lys Lys  Gly Glu Leu
    1085                 1090                 1095

Phe Asn  Gln Thr Ile Met Lys  Lys Gly Leu Gly Gln  His Pro Leu
    1100                 1105                 1110

Lys Lys  Glu Gly Pro Phe Ser  Asn Ile Ser Lys Tyr  Gly Gly Tyr
    1115                 1120                 1125

Asn Lys  Val Ser Ala Ala Tyr  Tyr Thr Leu Ile Glu  Tyr Glu Glu
    1130                 1135                 1140

Lys Gly  Asn Lys Ile Arg Ser  Leu Glu Thr Ile Pro  Leu Tyr Leu
    1145                 1150                 1155

Val Lys  Asp Ile Gln Lys Asp  Gln Asp Val Leu Lys  Ser Tyr Leu
    1160                 1165                 1170

Thr Asp  Leu Leu Gly Lys Lys  Glu Phe Lys Ile Leu  Val Pro Lys
    1175                 1180                 1185

Ile Lys  Ile Asn Ser Leu Leu  Lys Ile Asn Gly Phe  Pro Cys His
```

```
            1190                1195                1200

        Ile Thr  Gly Lys Thr Asn Asp  Ser Phe Leu Leu Arg  Pro Ala Val
            1205                1210                1215

        Gln Phe  Cys Cys Ser Asn Asn  Glu Val Leu Tyr Phe  Lys Lys Ile
            1220                1225                1230

        Ile Arg  Phe Ser Glu Ile Arg  Ser Gln Arg Glu Lys  Ile Gly Lys
            1235                1240                1245

        Thr Ile  Ser Pro Tyr Glu Asp  Leu Ser Phe Arg Ser  Tyr Ile Lys
            1250                1255                1260

        Glu Asn  Leu Trp Lys Lys Thr  Lys Asn Asp Glu Ile  Gly Glu Lys
            1265                1270                1275

        Glu Phe  Tyr Asp Leu Leu Gln  Lys Lys Asn Leu Glu  Ile Tyr Asp
            1280                1285                1290

        Met Leu  Leu Thr Lys His Lys  Asp Thr Ile Tyr Lys  Lys Arg Pro
            1295                1300                1305

        Asn Ser  Ala Thr Ile Asp Ile  Leu Val Lys Gly Lys  Glu Lys Phe
            1310                1315                1320

        Lys Ser  Leu Ile Ile Glu Asn  Gln Phe Glu Val Ile  Leu Glu Ile
            1325                1330                1335

        Leu Lys  Leu Phe Ser Ala Thr  Arg Asn Val Ser Asp  Leu Gln His
            1340                1345                1350

        Ile Gly  Gly Ser Lys Tyr Ser  Gly Val Ala Lys Ile  Gly Asn Lys
            1355                1360                1365

        Ile Ser  Ser Leu Asp Asn Cys  Ile Leu Ile Tyr Gln  Ser Ile Thr
            1370                1375                1380

        Gly Ile  Phe Glu Lys Arg Ile  Asp Leu Leu Lys Val
            1385                1390                1395
```

<210> 5
<211> 20
<212> DNA
<213> Mus musculus

<400> 5
ggagaggaca aaaaacaaga 20

<210> 6
<211> 23
<212> DNA
<213> Artificial Sequence

<220>
<223> mCherry

<400> 6
ggccacgagt tcgagatcga ggg 23

<210> 7
<211> 117
<212> DNA
<213> Artificial Sequence

<220>
<223> ssODN for mCherry

<400> 7

**agttcatgcg cttcaaggtg cacatggagg gctccgtgaa ttcataactt cgtatagcat 60**

**acattatacg aagttatcga gggcgagggc cgcccctacg agggcaccca gaccgcc 117**

<210> 8
<211> 50
<212> DNA
<213> Artificial Sequence

<220>
<223> ssODN for mCherry

<400> 8
cgtgaacggc cacgagttcg agatatcgag ggcgagggcg agggccgccc 50

<210> 9
<211> 20
<212> DNA
<213> Mus musculus

<400> 9
cagcaggtct tacccttcca 20

<210> 10
<211> 20
<212> DNA
<213> Mus musculus

<400> 10
tacaggggtt ggggacataa 20

<210> 11
<211> 20
<212> DNA
<213> Artificial Sequence

<220>
<223> Foward primer for mCherry

<400> 11
gagggcacta aggcagtcac 20

<210> 12
<211> 20
<212> DNA
<213> Artificial Sequence

<220>
<223> Reverse primer for mCherry

<400> 12
cccatggtct tcttctgcat 20

<210> 13
<211> 61
<212> DNA
<213> Mus musculus

<400> 13

**tgaatggaaa aggagctccc aggagaggac aaaaaacaag aaggaaaaac acctctgctc 60**

**a 61**

<210> 14
<211> 32
<212> DNA
<213> Artificial Sequence

<220>
<223> mCherry

<400> 14
gtgaacggcc acgagttcga gatcgagggc ga 32

<210> 15
<211> 41
<212> DNA
<213> Mus musculus

<400> 15
aggagctccc aggagaggac aaaaaacaag aaggaaaaac a 41

<210> 16
<211> 40
<212> DNA
<213> Artificial Sequence

<220>
<223> mCherry

<400> 16
tccgtgaacg gccacgagtt cgagatcgag ggcgagggcg 40

<210> 17
<211> 53
<212> DNA
<213> Artificial Sequence

<220>
<223> ssODN for mCherry

<400> 17
tccgtgaatt cataacttcg tatagcatac attatacgaa gttatcgagg gcg 53

## Description

TECHNICAL FIELD

This application claims the benefit of priority of the prior Japanese patent application (Japanese Patent Application No. 2015-031006).

The disclosure relates to a method of introducing mRNA encoding Cas9 protein (*Cas9* mRNA) into a non-human mammalian embryo by electroporation. The disclosure also relates to use of the method for preparing a non-human mammalian embryo expressing Cas9 protein, performing genome editing in a non-human mammalian embryo, preparing a non-human mammalian embryo whose genome is modified by genome editing, or preparing a genetically modified non-human animal.

BACKGROUND ART

Genetically modified animals are used for elucidating basic biological mechanisms or modeling human diseases in the fields including medical research and biology. As methods for creating genetically modified animals rapidly, processes utilizing artificial nucleases such as zinc-finger nucleases (ZFN), transcription activator-like effector nucleases (TALEN), and clustered regularly interspaced short palindromic repeat-associated system (CRISPR/Cas) have been attracted attention. These new techniques called "genome editing" have enabled to modify genomes in a wide variety of organisms without involving embryonic stem cells or induced pluripotent stem cells.

For creating a genetically modified animal by genome editing, DNA/RNA encoding an artificial nuclease has to be introduced into a pronuclear zygote. This has been achieved by microinjection, but microinjection involves disadvantage that a special skill is required for introducing DNA/RNA without disrupting the cell. Furthermore, the technique is inconvenient when numerous cells have to be treated at the same time, because DNA/RNA has to be microinjected to each pronuclear zygote one by one with a special device.

Nevertheless microinjection has been chosen in the most cases for introducing DNA/RNA into fertilized eggs. For example, linear DNAs to be inserted into genomes were microinjected into pronuclei, and circular plasmids or mRNAs for transient expression of desired genes were microinjected. Genome editing in mice, which has been established recently, is also achieved by microinjecting Cas9 mRNA and guide RNA (gRNA) or plasmids that encode the RNAs into cytoplasm or pronucleus of each embryo. The step of microinjection is rate-limiting in the generation of transgenic mice by genome editing since it requires a special skill and long time as stated above (Non-Patent Literature 1).

Electroporation is useful for introducing DNA/RNA of interest into a cell or tissue and has been applied for various organisms, for example fetal and postnatal mouse tissues including brain, testis, and muscle. However, electroporation has hardly been used for fertilized mouse eggs. It was exceptionally reported that short non-coding dsRNAs were introduced into fertilized mouse eggs by electroporation for knocking down endogenous genes (Non-Patent Literature 2), but this method is not practical because the eggs were treated with an acidic Tyrode's solution before the electroporation so that the zona pellucida was removed or thinned. The zona pellucida is essential for an embryo to be implanted and thus the treatment with the acidic Tyrode's solution is harmful. Furthermore, the method merely enabled the introduction of RNAs as short as less than 1000 bps. Another group reported that they performed electroporation without treating embryos with the acidic Tyrode's solution, but in their study only dsDNAs as short as about 500 bps were introduced into mouse embryos at the blastocyst stage (Non-Patent Literature 3).

Very recently introduction of *Cas9* mRNA and gRNA into fertilized rat eggs by electroporation without the treatment of the zona pellucida has been reported (Non-Patent Literature 4). However, in the study the efficiency of the genome editing was very low as shown in the results that genomes of less than 9% of the offspring were successfully modified, despite the fact that a large amount of mRNA at the concentration of 1000 to 2000 ng/μl was used.

REFERENCES

PATENT LITERATURE

[Patent Literature 1] US Patent No. 8697359

NON-PATENT LITERATURE

[Non-Patent Literature 1] Wang, H. et al., Cell 153, 910-918 (2013)
[Non-Patent Literature 2] Grabarek, JB. et al., Genesis 32:269-276 (2002)

[Non-Patent Literature 3] Scares, ML. et al., BMC Developmental Biology 2005, 5:28
[Non-Patent Literature 4] Kaneko, T. et al., Scientific Reports 4, 6382 (2014)
[Non-Patent Literature 5] Peng, H. et al., (2012) PLos ONE 7 (8): e43748
[Non-Patent Literature 6] Ohnishi Y. et al., Nucleic Acids Research, 2010, Vol.38, No.15 5141-5151
[Non-Patent Literature 7] Mazari, E. et al., Development (2014) 141, 2349-2359
[Non-Patent Literature 8] Yasue A., et al., Scientific Reports 4, 5705 (2014)
[Non-Patent Literature 9] Yang, H. et al., Cell 154, 1370-1379 (2013)
[Non-Patent Literature 10] Mashiko, D. et al., Scientific Reports 3, 3355 (2013)

WO2015/049897 describes a technology for modifying a target gene of a mammal by immersing a pronuclear stage mammalian zygote with an intact zona pellucida into a solution containing a pair of molecules of mRNA having a certain sequence, and performing electroporation treatment through application of multiple square-wave pulses in three steps with the total electric energy of a first electric pulse adjusted within a predetermined range.

[Non-patent Literature 2] Grabarek, J. et al., (2002) Efficient Delivery of dsRNA into Zona-Enclosed Mouse Oocytes and Preimplantation Embryos by Electroporation. GENESIS, Vol. 32, No. 4, pages 269-276 describes a study investigating the delivery of *c-mos* dsRNA into mouse oocytes and GFP dsRNA into mouse preimplantation embryos using electroporation.

[Non-Patent Literature 4] Kaneko, T. et al., (2014) Simple knockout by electroporation of engineered endonucleases into intact rat embryos. Scientific Reports. Vol. 4, No. 6382, Pages 1-5 describes a study investigating a method to produce knockout rats by introducing ZFN, TALEN and CRISPR/Cas mRNAs into intact embryos using electroporation.

[Non-Patent Literature 5] Peng, H. et al., (2012) Efficient Delivery of DNA and Morpholinos into Mouse Preimplantation Embryos by Electroporation. PLOS ONE, Vol. 7, No. 8, Pages 1-13 describes a study investigating an electroporation method of introducing DNA and morpholinos into mouse preimplantation embryos, which is tested on the endogenous gene *Oct4*.

## SUMMARY OF THE INVENTION

The present invention provides a method of introducing mRNA encoding Cas9 protein (*Cas9* mRNA) into a non-human mammalian embryo, comprising the steps of;

(a) placing a mixture of the non-human mammalian embryo and a solution comprising *Cas9* mRNA in the gap between a pair of electrodes, wherein the concentration of *Cas9* mRNA is at least 200 ng/μl, and
(b) applying a voltage to the electrodes for a voltage application duration,

wherein the voltage is at least 20 V per millimeter of the distance between the electrodes, and the voltage application duration is at least 15 msec; or
wherein the voltage is at least 30 V per millimeter of the distance between the electrodes, and the voltage application duration is at least 5 msec,
provided that the voltage is 20 to 40 V per millimeter of the distance between the electrodes, and the product of the voltage and the voltage application duration is not more than 990 Vmsec per millimeter of the distance between the electrodes.

The present invention also provides a method of preparing a non-human mammalian embryo expressing Cas9 protein, comprising introducing *Cas9* mRNA into a non-human mammalian embryo by the methods of the invention.

The present invention also provides a method of performing genome editing in a non-human mammalian embryo, comprising introducing *Cas9* mRNA and a further nucleic acid into the non-human mammalian embryo by the methods of the invention. The present invention also provides a method of preparing a non-human mammalian embryo whose genome is modified by genome editing, comprising introducing *Cas9* mRNA and a further nucleic acid into a non-human mammalian embryo by the methods of the invention.

The present invention also provides a method of preparing a genetically modified non-human animal, comprising transferring the non-human embryo obtained by the methods of the invention to a non-human recipient animal.

The inventors have found a suitable condition for introducing *Cas9* mRNA into a non-human mammalian embryo by electroporation.

Also described herein is a method of introducing mRNA encoding Cas9 protein (*Cas9* mRNA) into a non-human mammalian embryo, comprising the steps of;

(a) placing a mixture of the non-human mammalian embryo and a solution comprising *Cas9* mRNA in the gap between a pair of electrodes, and

(b) applying a voltage to the electrodes for a voltage application duration,

wherein the voltage and the voltage application duration achieve the efficiency of mRNA introduction (R) higher than the minimum required efficiency of mRNA introduction ($R_{min}$),
wherein R is calculated according to

Formula (I): $R = 0.0005 \times t^3 - 0.0057 \times t^2 + 0.2847 \times t$, when the voltage is about 20 to 30 V per millimeter of the distance between the electrodes;
Formula (II): $R = 0.0015 \times t^3 - 0.0191 \times t^2 + 0.9489 \times t$, when the voltage is about 30 to 40 V per millimeter of the distance between the electrodes;
Formula (III) : $R = 0.0005 \times t^3 + 0.0508 \times t^2 + 0.9922 \times t$, when the voltage is about 40 to 50 V per millimeter of the distance between the electrodes; or
Formula (IV): $R = 0.0078 \times t^3 - 0.1414 \times t^2 + 3.0103 \times t$, when the voltage is not less than about 50 V per millimeter of the distance between the electrodes;

in which t is the voltage application duration (msec),
wherein $R_{min}$ is calculated according to

$$\text{Formula (A): } R_{min} = 882 / c;$$

in which c is the concentration of *Cas9* mRNA (ng/µl),
provided that the voltage is about 20 to 55 V per millimeter of the distance between the electrodes, and the product of the voltage and the voltage application duration is not more than about 990 Vmsec per millimeter of the distance between the electrodes.

Also described herein is a method of preparing a non-human mammalian embryo expressing Cas9 protein, comprising the steps of;

(a) placing a mixture of a non-human mammalian embryo and a solution comprising *Cas9* mRNA in the gap between a pair of electrodes, and
(b) applying a voltage to the electrodes for a voltage application duration,

wherein the voltage and the voltage application duration achieve the efficiency of mRNA introduction (R) higher than the minimum required efficiency of mRNA introduction ($R_{min}$),
wherein R is calculated according to

Formula (I): $R = 0.0005 \times t^3 - 0.0057 \times t^2 + 0.2847 \times t$, when the voltage is about 20 to 30 V per millimeter of the distance between the electrodes;
Formula (II): $R = 0.0015 \times t^3 - 0.0191 \times t^2 + 0.9489 \times t$, when the voltage is about 30 to 40 V per millimeter of the distance between the electrodes;
Formula (III) : $R = 0.0005 \times t^3 + 0.0508 \times t^2 + 0.9922 \times t$, when the voltage is about 40 to 50 V per millimeter of the distance between the electrodes; or
Formula (IV): $R = 0.0078 \times t^3 - 0.1414 \times t^2 + 3.0103 \times t$, when the voltage is not less than about 50 V per millimeter of the distance between the electrodes;

in which t is the voltage application duration (msec),
wherein $R_{min}$ is calculated according to

$$\text{Formula (A): } R_{min} = 882 / c;$$

in which c is the concentration of *Cas9* mRNA (ng/µl),
provided that the voltage is about 20 to 55 V per millimeter of the distance between the electrodes, and the product of the voltage and the voltage application duration is not more than about 990 Vmsec per millimeter of the distance between the electrodes.

Also described herein is a method of performing genome editing in a non-human mammalian embryo, comprising the steps of;

(a) placing a mixture of the non-human mammalian embryo and a solution comprising *Cas9* mRNA and a further nucleic acid in the gap between a pair of electrodes, wherein the further nucleic acid is gRNA or a combination of CRISPR RNA (crRNA) and trans-activating CRISPR RNA (tracrRNA), and
(b) applying a voltage to the electrodes for a voltage application duration,

wherein the voltage and the voltage application duration achieve the efficiency of mRNA introduction (R) higher than the minimum required efficiency of mRNA introduction ($R_{min}$),
wherein R is calculated according to

Formula (I): $R = 0.0005 \times t^3 - 0.0057 \times t^2 + 0.2847 \times t$, when the voltage is about 20 to 30 V per millimeter of the distance between the electrodes;
Formula (II): $R = 0.0015 \times t^3 - 0.0191 \times t^2 + 0.9489 \times t$, when the voltage is about 30 to 40 V per millimeter of the distance between the electrodes;
Formula (III) : $R = 0.0005 \times t^3 + 0.0508 \times t^2 + 0.9922 \times t$, when the voltage is about 40 to 50 V per millimeter of the distance between the electrodes; or
Formula (IV): $R = 0.0078 \times t^3 - 0.1414 \times t^2 + 3.0103 \times t$, when the voltage is not less than about 50 V per millimeter of the distance between the electrodes;

in which t is the voltage application duration (msec),
wherein $R_{min}$ is calculated according to

$$\text{Formula (A): } R_{min} = 882 / c;$$

in which c is the concentration of *Cas9* mRNA (ng/μl),
provided that the voltage is about 20 to 55 V per millimeter of the distance between the electrodes, and the product of the voltage and the voltage application duration is not more than about 990 Vmsec per millimeter of the distance between the electrodes.

In a further example, described herein is a method of preparing a non-human mammalian embryo whose genome is modified by genome editing, comprising the steps of;

(a) placing a mixture of a non-human mammalian embryo and a solution comprising *Cas9* mRNA and a further nucleic acid in the gap between a pair of electrodes, wherein the further nucleic acid is gRNA or a combination of crRNA and tracrRNA, and
(b) applying a voltage to the electrodes for a voltage application duration,

wherein the voltage and the voltage application duration achieve the efficiency of mRNA introduction (R) higher than the minimum required efficiency of mRNA introduction ($R_{min}$),
wherein R is calculated according to

Formula (I): $R = 0.0005 \times t^3 - 0.0057 \times t^2 + 0.2847 \times t$, when the voltage is about 20 to 30 V per millimeter of the distance between the electrodes;
Formula (II): $R = 0.0015 \times t^3 - 0.0191 \times t^2 + 0.9489 \times t$, when the voltage is about 30 to 40 V per millimeter of the distance between the electrodes;
Formula (III) : $R = 0.0005 \times t^3 + 0.0508 \times t^2 + 0.9922 \times t$, when the voltage is about 40 to 50 V per millimeter of the distance between the electrodes; or
Formula (IV): $R = 0.0078 \times t^3 - 0.1414 \times t^2 + 3.0103 \times t$, when the voltage is not less than about 50 V per millimeter of the distance between the electrodes;

in which t is the voltage application duration (msec),
wherein $R_{min}$ is calculated according to

$$\text{Formula (A): } R_{min} = 882 / c;$$

in which c is the concentration of *Cas9* mRNA (ng/μl),
provided that the voltage is about 20 to 55 V per millimeter of the distance between the electrodes, and the product of the voltage and the voltage application duration is not more than about 990 Vmsec per millimeter of the distance between the electrodes.

According to the disclosure, *Cas9* mRNA can be introduced into a non-human mammalian embryo by electroporation.

BRIEF DESCRIPTION OF DRAWINGS

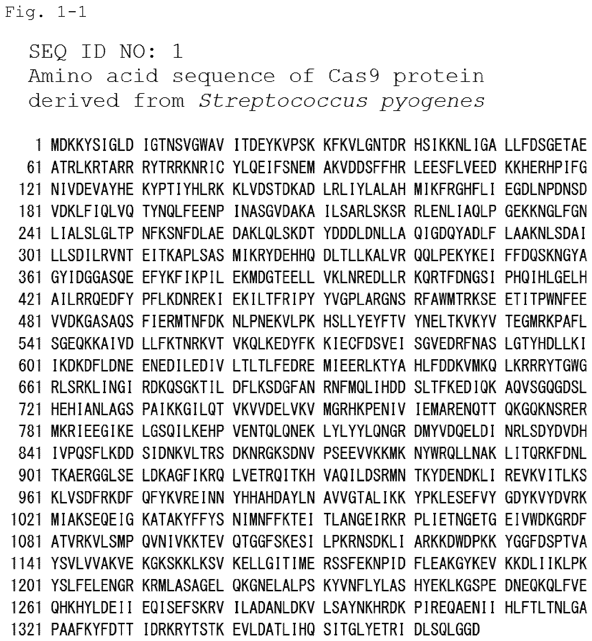

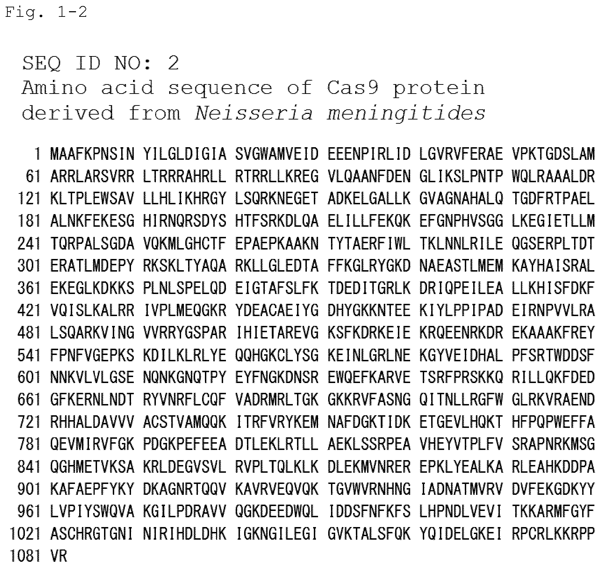

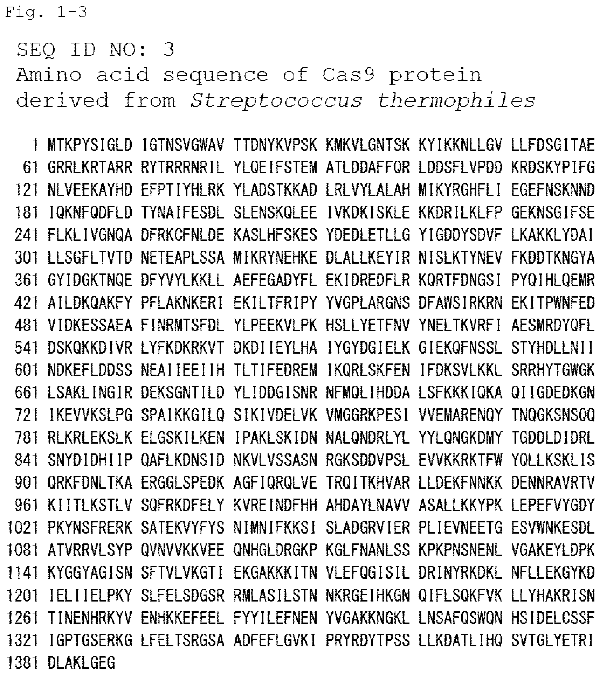

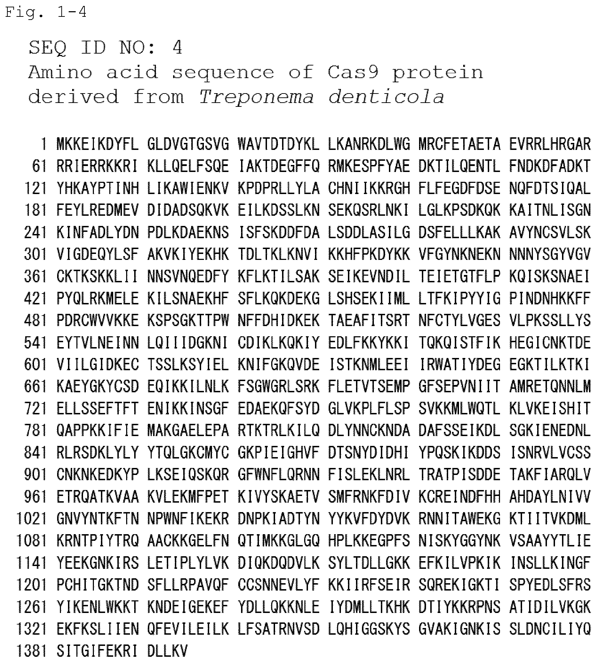

Fig. 1-1 shows the amino acid sequence of SEQ ID NO: 1.
Fig. 1-2 shows the amino acid sequence of SEQ ID NO: 2.
Fig. 1-3 shows the amino acid sequence of SEQ ID NO: 3.
Fig. 1-4 shows the amino acid sequence of SEQ ID NO: 4.
Fig. 2 illustrates the electroporation devise used in the examples.
Fig. 3 shows the fluorescence intensity of mCherry in embryos electroporated under various conditions and the survival rate of the embryos at the blastocyst stage.
Fig. 4 shows the efficiency of mRNA introduction for each voltage as a function of the voltage application duration, which is expected from the results shown in Fig. 3.
Fig. 5 shows the fluorescence intensity of mCherry in embryos electroporated with pulses of the both directions and the survival rate of the embryos at the blastocyst stage.
Fig. 6 illustrates CRISPR/Cas-mediated genome editing of *Fgf10* gene, wherein the RNAs were introduced by electroporation.
Fig. 7 shows the results of genome editing wherein high concentrations of *Cas9* mRNA were introduced by electroporation.
Fig. 8 illustrates the homology directed repair (HDR) of the *mCherry* gene, wherein the single-stranded oligodeoxynucleotide (ssODN) was introduced by electroporation.
Fig. 9 illustrates the HDR of the *mCherry* gene, wherein the ssODN was introduced by electroporation.

DETAILED DESCRIPTION

Unless otherwise defined, the terms used herein have the meaning as commonly understood to those skilled in the art in the fields including organic chemistry, medicine, pharmacology, developmental biology, cell biology, molecular biology, and microbiology. Definitions of several terms used herein are described below. The definitions herein take precedence over the general understanding.

In the disclosure when a value is accompanied with the term "about", the value is intended to include values within range of ± 10% of that value. A range defined by values of the both ends covers all values between the ends and the values of the ends. When a range is accompanied with the term "about", it is intended that the values of the both ends are accompanied with the term "about". For example, "about 20 to 30" means "20 ± 10% to 30 ± 10%".

Electroporation

As used herein, the term "electroporator" means a device that can generate an electric pulse. Any electroporator may be used as long as it enables steps (a) and (b) of the methods disclosed herein. Electroporators are available from manufacturers such as BioRad, BTX, BEX, Intracel, and Eppendorf.

As used herein, the term "electrode" includes any electrode that may be used for a conventional electroporation technique. For example, an electrode made of one or more metals such as platinum, gold, or aluminum may be used. Generally two electrodes are placed so that the distance between them is about 0.25 to 10 mm, for example about 0.5 to 4 mm or about 1 to 2 mm, providing a gap between the electrodes, in which a mixture of a non-human mammalian embryo and a solution comprising Cas9 mRNA can be placed. The two electrodes may be parts of a cuvette electrode, which also works as a container to receive the mixture. Electrodes are available from manufacturers such as BioRad, BTX, BEX, Intracel, and Eppendorf.

In the solution comprising *Cas9* mRNA, the concentration of *Cas9* mRNA is at least 200 ng/μl.

In some examples, the concentration of *Cas9* mRNA may be about 30 to 2000 ng/μl, about 50 to 1000 ng/μl, about 50 to 500 ng/μl, about 50 to 300 ng/μl, about 50 to 200 ng/μl, about 200 to about 1000 ng/μl, about 200 to 500 ng/μl, or about 200 to 300 ng/μl. In an embodiment, the solution contains *Cas9* mRNA at the concentration of 200 ng/μl.

Under a given electronic condition, the higher the mRNA concentration in the solution, the larger the amount of the mRNA introduced to an embryo.

Any solution that can be used for electroporation, i.e., any medium or buffer in which an embryo can survive during the electroporation, may be used for dissolving Cas9 mRNA to provide the solution used herein. For example, Opti-MEM I, PBS, HBS, HBSS, Hanks, and HCMF may be mentioned as such media or buffers. Preferably the solution contains no serum.

When the mixture of a non-human mammalian embryo and the solution comprising Cas9 mRNA is placed in the gap between the two electrodes, the embryo and the solution may be mixed first and then added to the gap, or the embryo and the solution may be separately added to the gap. The mixture is used in the volume that the mixture can fill the gap, for example, in the volume of about 1 to 50 μl, preferably about 1.5 to 15 μl, more preferably about 2 to 10 μl. In an embodiment, the volume of the mixture is about 5 μl.

In step (b), a voltage is applied to the electrodes for a voltage application duration, wherein the voltage is at least 20 V per millimeter of the distance between the electrodes, and the voltage application duration is at least 15 msec; or wherein the voltage is at least 30 V per millimeter of the distance between the electrodes, and the voltage application duration is at least 5 msec, provided that the voltage is 20 to 40 V per millimeter of the distance between the electrodes, and the product of the voltage and the voltage application duration is not more than 990 Vmsec per millimeter of the distance between the electrodes.

As described herein, in step (b), a voltage may be applied to the electrodes to achieve the efficiency of mRNA introduction (R) higher than the minimum required efficiency of mRNA introduction ($R_{min}$). $R_{min}$ depends on the concentration of *Cas9* mRNA and is calculated according to Formula (A) below:

$$\text{Formula (A): } R_{min} = 882 / c,$$

in which c is the concentration of *Cas9* mRNA (ng/μl).

The efficiency of mRNA introduction depends on the voltage and the voltage application duration. In some examples, the efficiency of mRNA introduction is calculated according to one of the following Formulae (I) to (IV) in which t is the voltage application duration (msec):

Formula (I): $R = 0.0005 \times t^3 - 0.0057 \times t^2 + 0.2847 \times t$, which is employed when the voltage is about 20 to 30 V per millimeter of the distance between the electrodes;

Formula (II): $R = 0.0015 \times t^3 - 0.0191 \times t^2 + 0.9489 \times t$, which is employed when the voltage is about 30 to 40 V per millimeter of the distance between the electrodes;

Formula (III) : $R = 0.0005 \times t^3 + 0.0508 \times t^2 + 0.9922 \times t$, which is employed when the voltage is about 40 to 50 V per millimeter of the distance between the electrodes;

Formula (IV): $R = 0.0078 \times t^3 - 0.1414 \times t^2 + 3.0103 \times t$, which is employed when the voltage is not less than 50 V per millimeter of the distance between the electrodes.

As described herein, when the voltage is about 30 V per millimeter of the distance between the electrodes, Formula (II) may be employed. As described herein, when the voltage is about 40 V per millimeter of the distance between the electrodes, Formula (III) may be employed. As described herein, when the voltage is about 50 V per millimeter of the distance between the electrodes, Formula (IV) may be employed.

As described herein, the efficiency of mRNA introduction (R) may be any value as long as it is not less than the minimum required efficiency of mRNA introduction ($R_{min}$) defined by the concentration of *Cas9* mRNA. For example, when the concentration of *Cas9* mRNA is 50 ng/μl, $R_{min}$ is 17.6, and then R may be at least 17.6, for example at least 25, preferably at least 27.1. For example, when the concentration of *Cas9* mRNA is 200 ng/μl, $R_{min}$ is 4.41, and then R may be at least 4.41, preferably at least 7.9, more preferably at least 14.7, and most preferably at least 27.1.

For example, when the concentration of *Cas9* mRNA is 50 ng/μl, $R_{min}$ is 17.6. In some examples, in order to achieve the efficiency of mRNA introduction (R) not less than the $R_{min}$ value, for example, per millimeter of the distance between the electrodes, a voltage of about 20 V is applied for at least about 31 msec, a voltage of about 30 V is applied for at least about 17 msec, a voltage of about 40 V is applied for at least about 11 msec, or a voltage of about 50 V is applied for at least about 7.5 msec; preferably, a voltage of about 20 V is applied for at least about 36 msec, a voltage of about 30 V is applied for at least about 21 msec, a voltage of about 40 V is applied for at least about 14 msec, or a voltage of about 50 V is applied for at least about 11 msec; more preferably, a voltage of about 20 V is applied for at least about 37 msec, a voltage of about 30 V is applied for at least about 22 msec, a voltage of about 40 V is applied for at least about 15 msec, or a voltage of about 50 V is applied for at least about 12 msec. In an embodiment, a voltage of about 30 V per millimeter of the distance between the electrodes is applied for about 21 msec.

For example, when the concentration of *Cas9* mRNA is 200 ng/μl, $R_{min}$ is 4.41. In order to achieve the efficiency of mRNA introduction (R) not less than the $R_{min}$ value, for example, per millimeter of the distance between the electrodes, a voltage of about 20 V is applied for at least about 15 msec, a voltage of about 30 V is applied for at least about 5 msec, or a voltage of about 40 V is applied for at least about 3.8 msec. Also described are methods in which a voltage of about 50 V is applied for at least about 1.6 msec. Preferably, a voltage of about 20 V is applied for at least about 21 msec, a voltage of about 30 V is applied for at least about 9 msec, or a voltage of about 40 V is applied for at least about 6 msec. Also described are methods in which a voltage of about 50 V is applied for at least about 3 msec. More preferably, a voltage of about 20 V is applied for at least about 29 msec, a voltage of about 30 V is applied for at least about 15 msec, or a voltage of about 40 V is applied for at least about 10 msec.. Also described are methods in which a voltage of about 50 V is applied for at least about 6 msec. Most preferably, a voltage of about 20 V is applied for at least about 37 msec, a voltage of about 30 V is applied for at least about 22 msec, or a voltage of about 40 V is applied for at least about 15 msec. Also described are methods in which a voltage of about 50 V is applied for at least about 12 msec. In an embodiment, a voltage of about 30 V per millimeter of the distance between the electrodes is applied for about 21 msec.

The efficiency of mRNA introduction is increased depending on the voltage and the voltage application duration. However, when the voltage is too high or the voltage application duration is too long, survival rate of the embryo tends to decrease. The voltage per millimeter of the distance between the electrodes should be 20 to 40 V, more preferably about 25 to 35 V, most preferably about 30 V. The voltage application duration is determined so that the product of the voltage and the voltage application duration per millimeter of the distance between the electrodes is not more than about 990 Vmsec, preferably not more than about 810 Vmsec, more preferably not more than about 630 Vmsec.

The voltage during the electroporation may be constant or varied. In an embodiment, the voltage is constant. A conventional square pulse electroporator can be used for generating a constant voltage.

In an embodiment, the voltage is applied as multiple pulses. For example, the voltage is applied as 2 to 15, 3 to 11, 5 to 9, or 6 to 8 pulses. In an embodiment, the voltage is applied as 7 pulses. The duration of each pulse is, for example, about 0.01 to 33 msec, about 0.5 to 15 msec, about 1 to 10 msec, or about 2 to 5 msec, for example, about 3 msec. The interval between each pulse is, for example, about 0.5 to 500 msec, preferably about 5 to 250 msec, more preferably about 10 to 150 msec, still preferably about 80 to 120 msec. In an embodiment, the interval between each pulse is about 97 msec. The duration and magnitude of each pulse may be same or different.

When the voltage is applied as multiple pulses, the direction of each pulse may be same or the direction of at least one pulse may be opposite to the others. When pulses of the both directions are applied, the pulses may be applied in any order. For example, sequential pulses of one direction may be applied and followed by sequential pulses of the opposite direction, pulses of the both directions may be applied in an alternate order, or pulses of the both directions may be applied in a random order.

As used herein, the term "pulse of the opposite direction" means, compared to a pulse generated by a pair of an anode and cathode, a pulse that is generated when the anode and cathode are interchanged. Similarly, when a pair of electrodes works as an anode and cathode to generate a voltage, the term "voltage of the opposite direction" means a voltage generated by interchanging the anode and cathode.

Also described are methods in which step (b) may be replaced with the following steps (c) and (d);

(c) applying a voltage to the electrodes for a voltage application duration,

wherein the voltage and the voltage application duration achieve the efficiency of mRNA introduction (R) higher than the minimum required efficiency of mRNA introduction ($R_{min}$),
wherein R is calculated according to

Formula (I): $R = 0.0005 \times t^3 - 0.0057 \times t^2 + 0.2847 \times t$, when the voltage is about 20 to 30 V per millimeter of the distance between the electrodes;
Formula (II): $R = 0.0015 \times t^3 - 0.0191 \times t^2 + 0.9489 \times t$, when the voltage is about 30 to 40 V per millimeter of the distance between the electrodes;
Formula (III) : $R = 0.0005 \times t^3 + 0.0508 \times t^2 + 0.9922 \times t$, when the voltage is about 40 to 50 V per millimeter of the distance between the electrodes; or
Formula (IV): $R = 0.0078 \times t^3 - 0.1414 \times t^2 + 3.0103 \times t$, when the voltage is not less than about 50 V per millimeter of the distance between the electrodes;

in which t is the voltage application duration (msec), wherein $R_{min}$ is calculated according to

$$\text{Formula (B)}: R_{min} = 441 / c;$$

in which c is the concentration of *Cas9* mRNA (ng/μl),

provided that the voltage is about 20 to 55 V per millimeter of the distance between the electrodes, the product of the voltage and the voltage application duration is not more than about 630 Vmsec, preferably not more than 540 Vmsec, per millimeter of the distance between the electrodes, and the voltage may be applied as two or more pulses; and

(d) applying a voltage of the opposite direction to the electrodes for a voltage application duration,

wherein the voltage and the voltage application duration achieve the efficiency of mRNA introduction (R) higher than the minimum required efficiency of mRNA introduction ($R_{min}$),
wherein R is calculated according to one of Formulae (I) to (IV);
wherein $R_{min}$ is calculated according to Formula (B);
provided that the voltage is about 20 to 55 V per millimeter of the distance between the electrodes, the product of the voltage and the voltage application duration is not more than about 630 Vmsec, preferably not more than 540 Vmsec, per millimeter of the distance between the electrodes, and the voltage may be applied as two or more pulses;
wherein when the voltage is applied as two or more pulses in steps (c) and (d), the two or more pulses may be applied as sequential pulses of one direction followed by sequential pulses of the opposite direction; pulses of the both directions in an alternate order; or pulses of the both directions in a random order.

In steps (c) and (d) the voltage and the voltage application duration may be determined as described for step (b) .

Genome editing

As used herein, "genome editing" means modifying one or more genes of a mammalian cell by using an artificial nuclease. One or both alleles are modified by the genome editing. A bacterial CRISPR/Cas9 system is used for the genome editing. Details of CRISPR/Cas systems are described in, for example, Wang, H. et al., Cell, 153, 910-918 (2013) and US Patent No. 8697359.

In general, genome editing with a CRISPR/Cas system requires Cas9 protein, an endonuclease, and gRNA. gRNA is a chimeric RNA in which bacterial crRNA and tracrRNA are combined. The crRNA is responsible for specificity to the target sequence and the tracrRNA works as a scaffold for Cas9 protein. When gRNA and Cas9 protein are expressed in a cell, the target sequence in the genome may be permanently modified.

The gRNA/Cas9 complex is recruited to the target sequence in the genome through complementary binding between the gRNA and the target sequence. The binding requires that a protospacer adjacent motif (PAM) is present immediately downstream of the target sequence in the genome. Cas9 protein localized to the target sequence cleaves the both strands of the genomic DNA, resulting in a double strand break (DSB). The DSB may be repaired through non-homologous end joining (NHEJ) pathway or homology directed repair (HDR) pathway. The NHEJ repair pathway frequently leads to insertion/deletion of at least a nucleotide (InDel) at the DSB site. The InDel may cause a frameshift and/or a stop codon, disrupting the open reading frame of the targeted gene. On the other hand, any desired mutation may be introduced to the target gene through the HDR pathway, because the HDR requires a DNA "repair template" and its sequence is copied to the cleaved genomic DNA.

Cas9 protein

Wild-type Cas9 proteins have two functional endonuclease domains, RuvC and HNH. The RuvC domain cleaves one strand of a double strand DNA and the HNH domain cleaves another strand. When the both domains are active, the Cas9 protein can generate the DSB in genomic DNA. Cas9 proteins having only one of the enzymatic activities have been developed. Such Cas9 proteins cleave only one strand of the target DNA. For example, the RuvC and HNH domains of the Cas9 protein derived from *Streptococcus pyogenes* are inactivated by D10A and H840A mutations, respectively.

Ability of Cas9 proteins to bind to a target DNA is independent from their ability to cleave the target DNA. Even if both of the RuvC and HNH domains are inactive and the Cas9 protein has no nuclease activity, the Cas9 protein still retains the ability to bind to the target DNA in the presence of gRNA. Accordingly, Cas9 proteins lacking nuclease activity (dCas9 proteins) may be used as a tool in molecular biology. For example, such dCas9 proteins may be used as a transcriptional regulator to activate or suppress expression of a gene through binding to a known transcriptional regulatory domain via gRNA. For example, if a dCas9 protein is fused with a transcriptional activator, it can activate transcription of the target gene. To the contrary, when only the dCas9 protein binds to the target sequence, the transcription may be suppressed. Expression of various genes may be regulated by targeting a sequence close to the promoter of the desired gene. Alternatively, in assays such as chromatin immunoprecipitation, genomic DNA may be purified by using a dCas9 protein fused with an epitope tag and a gRNA that targets any sequence in the genomic DNA. When a dCas9 protein fused with a fluorescent protein such as GFP or mcherry is used together with a gRNA that targets a desired sequence in genomic DNA, it may be used as a DNA label that can be detected in a living cell.

As used herein, the term "Cas9 protein" means a protein having an ability to bind to a DNA molecule in the presence of gRNA, including Cas9 proteins having both the RuvC and HNH nuclease activities and Cas9 proteins lacking either or both the nuclease activities. The DNA-binding activity and nuclease activity of Cas9 proteins may be measured, for example, by the method described in Samuel H. Sternberg et al., Nature 507, 62-67 (2014).

As used herein, the term "*Cas9* mRNA" means an mRNA encoding any one of the Cas9 proteins. The *Cas9* mRNA may have any nucleotide sequence as long as it is translated to an amino acid sequence of a Cas9 protein.

In an embodiment, a Cas9 protein derived from a bacterium having a CRISPR system is used. Bacteria known to have a CRISPR system include bacteria belonging to *Aeropyrum* sp., *Pyrobaculum* sp., *Sulfolobus* sp., *Archaeoglobus* sp., *Halocarcula* sp., *Methanobacteriumn* sp., *Methanococcus* sp., *Methanosarcina* sp., *Methanopyrus* sp., *Pyrococcus* sp., *Picrophilus* sp., *Thermoplasma* sp., *Corynebacterium* sp., *Mycobacterium* sp., *Streptomyces* sp., *Aquifex* sp., *Porphyromonas* sp., *Chlorobium* sp., *Thermus* sp., *Bacillus* sp., *Listeria* sp., *Staphylococcus* sp., *Clostridium* sp., *Thermoanaerobacter* sp., *Mycoplasma* sp., *Fusobacterium* sp., *Azoarcus* sp., *Chromobacterium* sp., *Neisseria* sp., *Nitrosomonas* sp., *Desulfovibrio* sp., *Geobacter* sp., *Micrococcus* sp., *Campylobacter* sp., *Wolinella* sp., *Acinetobacter* sp., *Erwinia* sp., *Escherichia* sp., *Legionella* sp., *Methylococcus* sp., *Pasteurella* sp., *Photobacterium* sp., *Salmonella* sp., *Xanthomonas* sp., *Yersinia* sp., *Treponema* sp., and *Thermotoga* sp. For example, a Cas9 protein derived from a bacterium such as *Streptococcus pyogenes, Neisseria meningitides, Streptococcus thermophiles,* or *Treponema denticola* is used.

In an embodiment, a Cas9 protein which is a fusion protein with at least one other protein or peptide may be used. Such proteins and peptides include, for example, fluorescent proteins, transcriptional factors, epitope tags, tags for protein purification, and nuclear localization signal peptides.

In an embodiment, a Cas9 protein may comprise an amino acid sequence having amino acid sequence identity at least about 80% with an amino acid sequence selected from SEQ ID NOs: 1 to 4 shown in Fig. 1 and have an ability to bind to DNA in the presence of gRNA and optionally the RuvC and/or HNH nuclease activity. For example, a Cas9 protein comprising or consisting of an amino acid sequence selected from SEQ ID NOs: 1 to 4 may be used. For example, a Cas9 protein comprising an amino acid sequence having amino acid sequence identity at least about 80% with the amino acid sequence of SEQ ID NO: 1 and having an ability to bind to DNA in the presence of gRNA and optionally the RuvC and/or HNH nuclease activity may be used. For example, a Cas9 protein comprising or consisting of the amino acid sequence of SEQ ID NO: 1 may be used. The amino acid sequences of SEQ ID NOs: 1 to 4 correspond to amino acid sequences of Cas9 proteins derived from *Streptococcus pyogenes, Neisseria meningitides,* Streptococcus *thermophiles,* and *Treponema denticola,* respectively.

In an embodiment, a Cas9 protein comprising an amino acid sequence having amino acid sequence identity at least about 80%, for example, at least about 85%, preferably at least about 90%, more preferably at least about 95%, still more preferably at least about 97%, still more preferably at least about 98%, still more preferably at least about 99%, still more preferably at least about 99.5% with an amino acid sequence selected from SEQ ID NOs: 1 to 4 may be used. The term "amino acid sequence identity" means the percentage of identical amino acid residues in given two amino acid sequences optimally aligned to each other. For example, 90% amino acid sequence identity means that 90% of total amino acid residues are identical between optimally aligned two amino acid sequences. Methods of aligning amino acid sequences and calculating amino acid sequence identity are known to those skilled in the art. For example, programs such as BLAST may be used.

*Cas9* mRNA may be obtained by cloning a DNA coding an amino acid sequence of a desired Cas9 protein into a vector suitable for in vitro transcription and performing in vitro transcription. Vectors suitable for in vitro transcription are known to those skilled in the art. In vitro transcription vectors that contain a cloned DNA encoding a Cas9 protein are also known and include, for example, pT7-Cas9 available from Origene. Methods of in vitro transcription are known to those skilled in the art.

In an embodiment, the solution comprising *Cas9* mRNA may contain at least one further nucleic acid and the nucleic acid may be introduced to an embryo together with the *Cas9* mRNA. The further nucleic acid may be, for example, gRNA, crRNA, tracrRNA or ssODN. For example, gRNA alone, combination of crRNA and tracrRNA, combination of gRNA and ssODN, or combination of crRNA, tracrRNA and ssODN may be used.

The concentration ratio of gRNA to *Cas9* mRNA may be 1:20 to 1:1, for example 1:2, in weight. For example, the solution may contain 200 ng/μl *Cas9* mRNA and 100 ng/μl gRNA. The concentration ratio of crRNA to tracrRNA to *Cas9* mRNA may be 1:1:20 to 1:1:1, for example 1:1:2, in weight. For example, the solution may contain 200 ng/μl *Cas9* mRNA, 100 ng/μl crRNA and 100 ng/μl tracrRNA. The concentration of ssODN in the solution may be 200 to 1000 ng/μl, for example, 600 ng/μl.

gRNA

Genome editing requires a target-specific gRNA. As used herein, the term "guide RNA" or "gRNA" means a synthetic single-strand RNA comprising a fusion of crRNA and tracrRNA. The crRNA and tracrRNA may be linked via a linker. Cas9 protein can bind to a target sequence in genomic DNA in the presence of gRNA specific for the target sequence.

crRNA is derived from an endogenous bacterial RNA and is responsible for sequence specificity of gRNA. crRNA comprising a target sequence present in genomic DNA or the sequence compliment thereto is used herein. The target sequence is selected so that the sequence is present immediately upstream of a protospacer adjacent motif (PAM) in the genomic DNA. The target sequence may be present in either strand of the genomic DNA. Many tools are available for selecting a target sequence and/or designing gRNA, and lists of target sequences which are predicted for various genes in various species may be obtained. For example, Feng Zhang lab's Target Finder, Michael Boutros lab's Target Finder (E-CRISP), RGEN Tools: Cas-OFFinder, CasFinder: Flexible algorithm for identifying specific Cas9 targets in genomes, and CRISPR Optimal Target Finder, may be mentioned.

The PAM sequence is present immediately downstream of the target sequence in the genomic DNA, but not present immediately downstream of the target sequence in the gRNA. Cas9 proteins can bind to any DNA sequence as long as the DNA has the PAM sequence immediately downstream of the target sequence. The exact sequence of the PAM is dependent upon the bacterial species from which the Cas9 protein is derived. One of the most widely used Cas9 proteins is derived from *Streptococcus pyogenes* and the corresponding PAM sequence is NGG present immediately downstream of the 3' end of the target sequence. PAM sequences of various bacterial species are known, for example, *Neisseria meningitides:* NNNNGATT, *Streptococcus thermophiles:* NNAGAA, *Treponema* denticola: NAAAAC. In these sequences, N represents any one of A, T, G, and C.

tracrRNA hybridizes to a part of crRNA to form a hairpin loop structure. The structure is recognized by Cas9 protein and a complex of crRNA, tracrRNA and Cas9 protein is formed. Thus tracrRNA is responsible for the ability of gRNA to bind to Cas9 protein. tracrRNA is derived from an endogenous bacterial RNA and has a sequence intrinsic to the bacterial species. tracrRNA derived from the bacterial species known to have a CRISPR system listed above may be used herein. Preferably, tracrRNA and Cas9 protein derived from the same species are used. For example, tracrRNA derived from *Streptococcus pyogenes, Neisseria meningitides, Streptococcus thermophiles,* or *Treponema denticola* may be used.

gRNA may be obtained by cloning a DNA having a desired gRNA sequence into a vector suitable for in vitro transcription and performing in vitro transcription. Vectors suitable for in vitro transcription are known to those skilled in the art. In vitro transcription vectors that comprise a sequence corresponding to gRNA with no target sequence are also known in the art. gRNA may be obtained by inserting a synthesized oligonucleotide of a target sequence into such vector and performing in vitro transcription. Such vectors include, for example, pUC57-sgRNA expression vector, pCFD1-dU6:1gRNA, pCFD2-dU6:2gRNA pCFD3-dU6:3gRNA, pCFD4-U6:1_U6:3tandemgRNAs, pRB17, pMB60, DR274, SP6-sgRNA-scaffold, pT7-gRNA, DR274, and pUC57-Simple-gRNA backbone available from Addgene, and pT7-Guide-IVT available from Origene. Methods of in vitro transcription are known to those skilled in the art.

Combination of crRNA and tracrRNA may be used in place of gRNA. When the combination is used, the crRNA and tracrRNA are separate RNA molecules and the weight ratio of crRNA to tracrRNA may be 1:10 to 10:1, for example 1:1.

Homology Directed Repair (HDR)

Combination of CRISPR/Cas system with HDR can modify one or more desired nucleotides in a target sequence. In order to utilize the HDR for gene editing, a DNA repair template containing a desired sequence is necessary. In an embodiment, the DNA repair template is a single-stranded oligodeoxynucleotide (ssODN). ssODN has homology to the sequences immediately upstream and downstream of the DSB. The length and binding position of each homology region is dependent on the size of the change to be introduced. In the presence of a suitable template, the HDR can modify the desired nucleotide at the position of the DSB made by Cas9 protein. ssODN is designed so that the modified gene is not cleaved by the Cas9 protein. This means that ssODN should not contain the PAM sequence immediately downstream of the target sequence. For example, the sequence modified by ssODN is not cleaved by Cas9 protein when the ssODN has a nucleotide sequence different from the PAM sequence at the positon corresponding to the PAM sequence. Details of methods of designing ssODNs are described in, for example, Yang, H. et al., Cell, 154(6), 1370-9 (2013). In general, ssODN is introduced into a cell together with gRNA and *Cas9* mRNA.

As used herein, the term "introducing mRNA encoding Cas9 protein into an embryo" or "introducing *Cas9* mRNA into an embryo" means introducing *Cas9* mRNA to an embryo by electroporation at the amount that enables expression of Cas9 protein in the embryo or at least one cell derived from the embryo. Preferably, *Cas9* mRNA is introduced at the amount that enables genome editing of at least one target gene in the genome of the embryo or at least one cell derived from the embryo in the presence of gRNA.

In an embodiment, it is confirmed that the genome editing has occurred. Whether the genome editing has occurred can be confirmed by various methods known in the art. For example, when the phenotype of the target gene is known, change of the phenotype may be detected. Alternatively, the region comprising the target sequence in the genomic DNA of the embryo or at least one cell derived from the embryo may be sequenced. In the case of HDR, a restriction enzyme site may be incorporated to ssODN and the restriction fragment length polymorphism (RFLP) may be detected. These methods are well known in the art.

As used herein, the term "mammalian" or "mammal" means any organism that is classified in the Mammalia. The mammal includes, for example, primates (e.g., monkey), rodents (e.g., mouse, rat, guinea pig, hamster), cattle, pig, sheep, goat, horse, dog, cat, and rabbit. In an embodiment, the mammal is a rodent. In an embodiment, the mammal is a mouse.

As used herein, the term "embryo" means an egg or embryo after a fertilization event, including a fertilized egg (one-cell stage) and early embryos from the two-cell stage to the blastocyst stage. The fertilization may occur in vivo or in vitro. Embryos may be stored frozen prior to or after the fertilization. Methods of preparing, culturing and storing embryos are known in the art. Preferably, prior to the electroporation, embryos are washed with a solution for the electroporation to remove the culture medium.

In an embodiment, the embryo is at the one-cell stage to the morula stage, preferably at the one-cell stage to the eight-cell stage, more preferably at the one-cell stage to the four-cell stage, still more preferably at the one-cell stage or the two-cell stage, for example, at the one-cell stage. In an embodiment, the electroporation is performed at least about 6 hours, preferably at least about 9 hours, more preferably at least about 12 hours after the fertilization. In an embodiment, the electroporation is performed about 6 to 18 hours, preferably about 9 to 15 hours, more preferably about 11 to 13 hours, for example about 12 hours after the fertilization. Usually, embryos have a protective membrane called zona pellucida, which can be removed or thinned e.g. by treatment with an acidic Tyrode's solution. The zona pellucida may be removed or thinned, but this is not an indispensable step herein. Preferably, the zona pellucida is not removed or thinned.

In an embodiment, the electroporated embryo is cultured and the survival of the embryo is confirmed. Methods of culturing an embryo are well known to those skilled in the art. Survival of the embryo can be confirmed by observing that at least one cell division occurred in the embryo after the electroporation.

In an embodiment, a non-human mammalian embryo whose genome is modified by genome editing may be obtained. Another embodiment provides a method of preparing a genetically modified non-human animal comprising the step of transferring the obtained non-human embryo to a recipient non-human animal. The recipient non-human animal is usually a pseudopregnant female of the same animal species as the embryo. The embryo is usually implanted to the fallopian tube. Depending on the developmental stage of the embryo, it may be implanted to the uterus. The recipient non-human animal implanted with the non-human embryo delivers a genetically modified non-human animal. Methods of preparing a genetically modified non-human animal are known to those skilled in the art. For example, the method described in Manipulating the Mouse Embryo: A Laboratory Manual, Fourth Edition (Cold Spring Harbor Press), may be used.

In an embodiment, the following steps are employed; (a) placing a mixture of a non-human mammalian embryo and a solution comprising at least about 200 ng/μl *Cas9* mRNA in the gap between a pair of electrodes, and

(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 990 Vmsec per millimeter of the distance between the electrodes.

In an embodiment, the following steps are employed;

(a) placing a mixture of a non-human mammalian embryo and a solution comprising at least about 200 ng/μl *Cas9* mRNA in the gap between a pair of electrodes, and

(b) applying a voltage of at least about 30 V per millimeter of the distance between the electrodes for at least about 21 msec to the electrodes,

provided that the product of the voltage and the voltage application duration is not more than about 990 Vmsec per millimeter of the distance between the electrodes.

Also described is a method where the following steps are employed;

(a) placing a mixture of a non-human mammalian embryo and a solution comprising at least about 50 ng/μl *Cas9* mRNA in the gap between a pair of electrodes, and

(b) applying a voltage of at least about 30 V per millimeter of the distance between the electrodes for at least about 21 msec to the electrodes,

provided that the product of the voltage and the voltage application duration is not more than about 990 Vmsec per millimeter of the distance between the electrodes.

In an embodiment, the following steps are employed;

(a) placing a mixture of a non-human mammalian embryo and a solution comprising at least about 200 ng/μl *Cas9* mRNA in the gap between a pair of electrodes, and
(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 630 Vmsec per millimeter of the distance between the electrodes.
In an embodiment, the following steps are employed;

(a) placing a mixture of a non-human mammalian embryo and a solution comprising at least about 200 ng/μl *Cas9* mRNA in the gap between a pair of electrodes, and
(b) applying a voltage of at least about 30 V per millimeter of the distance between the electrodes for at least about 21 msec to the electrodes

provided that the product of the voltage and the voltage application duration is not more than about 630 Vmsec per millimeter of the distance between the electrodes.
Also described is a method where the following steps are employed;

(a) placing a mixture of a non-human mammalian embryo and a solution comprising at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.
In an embodiment, the following steps are employed; (a) placing a mixture of a non-human mammalian embryo at the one-cell stage and a solution comprising at least about 200 ng/μl *Cas9* mRNA in the gap between a pair of electrodes, and (b) applying a voltage of at least 20 V per millimeter of the distance between the electrodes for at least about 15 msec or a voltage of at least about 30 V per millimeter of the distance between the electrodes for at least about 9 msec to the electrodes,
provided that the product of the voltage and the voltage application duration is not more than about 630 Vmsec per millimeter of the distance between the electrodes.
In an embodiment, the following steps are employed; (a) placing a mixture of a non-human mammalian embryo at the one-cell stage and a solution comprising at least about 200 ng/μl *Cas9* mRNA in the gap between a pair of electrodes, and (b) applying a voltage of at least about 30 V per millimeter of the distance between the electrodes for at least about 21 msec to the electrodes, provided that the product of the voltage and the voltage application duration is not more than about 630 Vmsec per millimeter of the distance between the electrodes.
Also described is a method where the following steps are employed;

(a) placing a mixture of a non-human mammalian embryo at the one-cell stage and a solution comprising at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.
In an embodiment, the following steps are employed;

(a) placing a mixture of a non-human mammalian embryo and a solution comprising gRNA and at least about 200 ng/μl *Cas9* mRNA in the gap between a pair of electrodes, and
(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 630 Vmsec per millimeter of the distance between the electrodes.

In an embodiment, the following steps are employed; (a) placing a mixture of a non-human mammalian embryo and a solution comprising crRNA, tracrRNA and at least about 200 ng/μl *Cas9* mRNA in the gap between a pair of electrodes, and (b) applying a voltage of at least 20 V per millimeter of the distance between the electrodes for at least about 15 msec or a voltage of at least about 30 V per millimeter of the distance between the electrodes for at least about 9 msec to the electrodes,
provided that the product of the voltage and the voltage application duration is not more than about 630 Vmsec per millimeter of the distance between the electrodes.

In an embodiment, the following steps are employed;

(a) placing a mixture of a non-human mammalian embryo and a solution comprising gRNA, ssODN and at least about 200 ng/μl *Cas9* mRNA in the gap between a pair of electrodes, and
(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo and a solution comprising crRNA, tracrRNA, ssODN and at least about 200 ng/μl *Cas9* mRNA in the gap between a pair of electrodes, and
(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo and a solution comprising gRNA and at least about 200 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

In an embodiment, the following steps are employed; (a) placing a mixture of a non-human mammalian embryo and a solution comprising crRNA, tracrRNA and at least about 200 ng/μl *Cas9* mRNA in the gap between a pair of electrodes, and (b) applying a voltage of at least about 30 V per millimeter of the distance between the electrodes for at least about 21 msec to the electrodes,
provided that the product of the voltage and the voltage application duration is not more than about 630 Vmsec per millimeter of the distance between the electrodes.

In an embodiment, the following steps are employed;

(a) placing a mixture of a non-human mammalian embryo and a solution comprising gRNA, ssODN and at least about 200 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo and a solution comprising crRNA, tracrRNA, ssODN and at least about 200 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

Also described is a method where the following steps are employed;

(a) placing a mixture of a non-human mammalian embryo and a solution comprising gRNA and at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

Also described is a method where the following steps are employed;

(a) placing a mixture of a mammalian embryo and a solution comprising crRNA, tracrRNA and at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

Also described is a method where the following steps are employed;

(a) placing a mixture of a non-human mammalian embryo and a solution comprising gRNA, ssODN and at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

Also described is a method where the following steps are employed;

(a) placing a mixture of a non-human mammalian embryo and a solution comprising crRNA, tracrRNA, ssODN and at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo at the one-cell stage and a solution comprising gRNA and at least about 200 ng/μl *Cas9* mRNA in the gap between a pair of electrodes, and
(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo at the one-cell stage and a solution comprising crRNA, tracrRNA and at least about 200 ng/μl *Cas9* mRNA in the gap between a pair of electrodes, and
(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo at the one-cell stage and a solution comprising gRNA, ssODN and at least about 200 ng/μl *Cas9* mRNA in the gap between a pair of electrodes, and
(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo at the one-cell stage and a solution comprising crRNA, tracrRNA, ssODN and at least about 200 ng/μl *Cas9* mRNA in the gap between a pair of electrodes, and
(b) applying a voltage of at least 20 V per millimeter of the distance between the electrodes for at least about 15 msec or a voltage of at least about 30 V per millimeter of the distance between the electrodes for at least about 9 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo at the one-cell stage and a solution comprising gRNA and at least about 200 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo at the one-cell stage and a solution comprising crRNA, tracrRNA and at least about 200 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo at the one-cell stage and a solution comprising gRNA, ssODN and at least about 200 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

In an embodiment, the following steps are employed;

(a) placing a mixture of a non-human mammalian embryo at the one-cell stage and a solution comprising crRNA, tracrRNA, ssODN and at least about 200 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

Also described is a method where the following steps are employed;

(a) placing a mixture of a non-human mammalian embryo at the one-cell stage and a solution comprising gRNA and at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

Also described is a method where the following steps are employed;

(a) placing a mixture of a non-human mammalian embryo at the one-cell stage and a solution comprising crRNA, tracrRNA and at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

Also described is a method where the following steps are employed;

(a) placing a mixture of a non-human mammalian embryo at the one-cell stage and a solution comprising gRNA, ssODN and at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes.

Also described is a method where the following steps are employed;

(a) placing a mixture of a non-human mammalian embryo at the one-cell stage and a solution comprising crRNA, tracrRNA, ssODN and at least about 50 ng/μl *Cas9* mRNA in the gap between a pair of electrodes, and
(b) applying a voltage of at least about 30 V per millimeter of the distance between the electrodes for at least about 21 msec to the electrodes,

provided that the product of the voltage and the voltage application duration is not more than about 630 Vmsec per millimeter of the distance between the electrodes. distance between the electrodes; or

$$\text{Formula (IV): } R = 0.0078 \times t^3 - 0.1414 \times t^2 + 3.0103 \times t,$$

when the voltage is not less than about 50 V per millimeter of the distance between the electrodes;
in which t is the voltage application duration (msec),
wherein $R_{min}$ is calculated according to

$$\text{Formula (A): } R_{min} = 882 / c;$$

in which c is the concentration of *Cas9* mRNA (ng/μl),
provided that the voltage is about 20 to 55 V per millimeter of the distance between the electrodes, and the product of the voltage and the voltage application duration is not more than about 990 Vmsec per millimeter of the distance between the electrodes.

[2] The method according to item [1], wherein the voltage is about 20 to 40 V per millimeter of the distance between the electrodes.

[3] The method according to item [1] or [2], wherein the voltage is about 25 to 35 V per millimeter of the distance between the electrodes.

[4] The method according to any one of items [1] to [3], wherein the voltage is about 30 V per millimeter of the distance between the electrodes.

[5] The method according to any one of items [1] to [4], wherein the mRNA concentration is about 50 to 1000 ng/μl.

[6] The method according to any one of items [1] to [5], wherein the mRNA concentration is about 50 to 200 ng/μl.

[7] The method according to any one of items [1] to [6], wherein the mRNA concentration is at least about 50 ng/μl, and the efficiency of mRNA introduction is at least about 25.

[8] The method according to item [7], wherein the voltage is at least about 20 V per millimeter of the distance between the electrodes, and the voltage application duration is at least about 36 msec.

[9] The method according to item [7], wherein the voltage is at least about 30 V per millimeter of the distance between the electrodes, and the voltage application duration is at least about 21 msec.

[10] The method according to item [7], wherein the voltage is at least about 40 V per millimeter of the distance between the electrodes, and the voltage application duration is at least about 14 msec.

[11] The method according to item [7], wherein the voltage is at least about 50 V per millimeter of the distance between the electrodes, and the voltage application duration is at least about 11 msec.

[12] The method according to any one of items [1] to [6], wherein the mRNA concentration is at least about 200 ng/μl, and the efficiency of mRNA introduction is at least about 4.41.

[13] The method according to item [12], wherein the voltage is at least about 20 V per millimeter of the distance between the electrodes, and the voltage application duration is at least about 15 msec.

[14] The method according to item [12], wherein the voltage is at least about 30 V per millimeter of the distance between the electrodes, and the voltage application duration is at least about 5 msec.

[15] The method according to item [12], wherein the voltage is at least about 30 V per millimeter of the distance between the electrodes, and the voltage application duration is at least about 9 msec.

[16] The method according to item [12], wherein the voltage is at least about 40 V per millimeter of the distance between the electrodes, and the voltage application duration is at least about 3.8 msec.

[17] The method according to item [12], wherein the voltage is at least about 50 V per millimeter of the distance between the electrodes, and the voltage application duration is at least about 1.6 msec.

[18] The method according to any one of items [1] to [17], wherein the voltage is constant.

[19] The method according to any one of items [1] to [18], wherein the product of the voltage and the voltage application duration is not more than about 630 Vmsec per millimeter of the distance between the electrodes.

[20] The method according to any one of items [1] to [19], wherein the voltage is applied as 2 to 15 pulses.

[21] The method according to any one of items [1] to [20], wherein the voltage is applied as 5 to 11 pulses.

[22] The method according to item [20] or [21], wherein the interval between each pulse is about 10 to 150 msec.

[23] The method according to any one of items [20] to [22], wherein the pulses are applied in one direction.

[24] The method according to any one of items [20] to [22], wherein at least one pulse is applied in a direction opposite to the others.

[25] A method of introducing *Cas9* mRNA into a non-human mammalian embryo, comprising the steps of;

(a) placing a mixture of the non-human mammalian embryo and a solution comprising *Cas9* mRNA in the gap between a pair of electrodes,
(c) applying a voltage to the electrodes for a voltage application duration,

wherein the voltage and the voltage application duration achieve the efficiency of mRNA introduction (R) higher than the minimum required efficiency of mRNA introduction ($R_{min}$),
wherein R is calculated according to

Formula (I): $R = 0.0005 \times t^3 - 0.0057 \times t^2 + 0.2847 \times t$, when the voltage is about 20 to 30 V per millimeter of the distance between the electrodes;
Formula (II): $R = 0.0015 \times t^3 - 0.0191 \times t^2 + 0.9489 \times t$, when the voltage is about 30 to 40 V per millimeter of the distance between the electrodes;
Formula (III): $R = 0.0005 \times t^3 + 0.0508 \times t^2 + 0.9922 \times t$, when the voltage is about 40 to 50 V per millimeter of the distance between the electrodes; or
Formula (IV): $R = 0.0078 \times t^3 - 0.1414 \times t^2 + 3.0103 \times t$, when the voltage is not less than about 50 V per millimeter of the distance between the electrodes;

in which t is the voltage application duration (msec),
wherein $R_{min}$ is calculated according to

$$\text{Formula (B): } R_{min} = 441 / c;$$

in which c is the concentration of *Cas9* mRNA (ng/μl),
provided that the voltage is about 20 to 55 V per millimeter of the distance between the electrodes, the product of the voltage and the voltage application duration is not more than about 630 Vmsec per millimeter of the distance between the electrodes, and the voltage may be applied as two or more pulses; and

(d) applying a voltage of the opposite direction to the electrodes for a voltage application duration,

wherein the voltage and the voltage application duration achieve the efficiency of mRNA introduction (R) higher than the minimum required efficiency of mRNA introduction ($R_{min}$),
wherein R is calculated according to one of Formulae (I) to (IV);
wherein $R_{min}$ is calculated according to Formula (B);
provided that the voltage is about 20 to 55 V per millimeter of the distance between the electrodes, the product of the voltage and the voltage application duration is not more than about 630 Vmsec per millimeter of the distance between the electrodes, and the voltage may be applied as two or more pulses;
wherein when the voltage is applied as two or more pulses in steps (c) and (d), the two or more pulses may be applied as sequential pulses of one direction followed by sequential pulses of the opposite direction; pulses of the both directions in an alternate order; or pulses of the both directions in a random order.

[26] The method according to any one of items [1] to [25], wherein the non-human embryo is at the one-cell stage or the two-cell stage.

[27] The method according to any one of items [1] to [26], wherein the non-human embryo is at the one-cell stage.

[28] The method according to any one of items [1] to [27], wherein the electroporation is performed about 12 hours after the fertilization.

[29] The method according to any one of items [1] to [28], wherein the embryo is a rodent embryo.

[30] The method according to any one of items [1] to [29], wherein the embryo is a mouse embryo.

[31] The method according to any one of items [1] to [30], wherein the Cas9 protein comprises an amino acid sequence having at least about 90% amino acid sequence identity with the amino acid sequence of any one of SEQ ID NOs: 1 to 4 and has an ability to bind to DNA in the presence of gRNA.

[32] The method according to any one of items [1] to [31], wherein the Cas9 protein has RuvC and/or HNH nuclease activity.

[33] The method according to any one of items [1] to [32], wherein the Cas9 protein comprises the amino acid sequence of any one of SEQ ID NOs: 1 to 4.

[34] The method according to any one of items [1] to [33], wherein the Cas9 protein comprises the amino acid sequence of SEQ ID NO: 1.

[35] The method according to any one of items [1] to [34], wherein the solution comprises at least one further nucleic acid and the nucleic acid is introduced to the non-human embryo together with the *Cas9* mRNA.

[36] The method according to item [35], wherein the nucleic acid is gRNA, or combination of crRNA and tracrRNA.

[37] The method according to item [35] or [36], wherein the nucleic acid is gRNA.

[38] The method according to item [36] or [37], wherein the solution further comprises ssODN.

[39] The method according to any one of items [1] to [38], further comprising culturing the electroporated non-human embryo and confirming the survival of the embryo.

[40] A method of preparing a mammalian embryo expressing Cas9 protein, comprising introducing *Cas9* mRNA into a non-human mammalian embryo by the method according to any one of items [1] to [39].

[41] A method of performing genome editing in a non-human mammalian embryo, comprising introducing *Cas9* mRNA and a further nucleic acid into the non-human mammalian embryo by the method according to any one of items [35] to [38].

[42] The method according to item [41], further comprising confirming whether the genome editing has occurred.

[43] A method of preparing a non-human mammalian embryo whose genome is modified by genome editing, comprising introducing *Cas9* mRNA

According to the disclosure, *Cas9* mRNA can be introduced into a non-human mammalian embryo efficiently and quickly by electroporation. Electroporation advantageously results in high survival rate of the non-human embryos and does not require any special skill and much time. For example, even a skilled technician needs at least one hour in order to introduce mRNA into each cytoplasm or pronucleus of 100 embryos by microinjection, while electroporation easily enables the same within few minutes. Furthermore, a device for electroporation is usually cheaper than that for microinjection. Thus, the disclosure is useful for improving the efficiency, speed and cost of CRISPR/Cas9-mediated genome editing and thus generation of a genetically modified non-human animal.

EXAMPLES

Materials and Methods

mRNA and gRNA preparation

pCS2-mCherry was kindly provided by Dr. Noriyuki Kinoshita (NIBB, Japan). hCas9 plasmid (pX330) was purchased from Addgene (Cambridge, MA, USA). *hCas9* gene was excised from pX330, then placed downstream of SP6 promoter in pSP64 vector (Promega) (pSP64-hCas9) and used for RNA synthesis. pCS2-mCherry and pSP64-hCas9 were linearized by digestion with NotI and SalI, respectively, and used as templates for *mCherry* and *hCas9* mRNA synthesis using an in vitro RNA transcription kit (mMESSAGE mMACHINE SP6 Transcription Kit, Ambion, Austin, TX, USA) .

A pair of oligos targeting *Fgf10* or *mCherry* was annealed and inserted into BsaI site of pDR274 vector (Addgene). The sequences of the oligos were as follows: *FgflO* (5' -GGAGAGGACAAAAAACAAGA-3' (SEQ ID NO: 5) and the complementary sequence) and mCherry (5'-GGCCACGAGTTCGAGATCGAGGG-3' (SEQ ID NO: 6) and the complementary sequence). After digestion with DraI, gRNAs were synthesized using the MEGAshortscript T7 Transcription Kit (Ambion, Austin, TX, USA).

The synthesized RNAs, mRNA and gRNAs, were purified by phenol-chloroform-isoamylalcohol extraction and isopropanol precipitation. The precipitated RNAs were dissolved in Opti-MEM I at 2-4 μg/μl, and stored at -20°C until use. The RNAs were quantified by absorption spectroscopy and agarose gel electrophoresis. ssODNs were purchased from Sigma in dry form, dissolved in Opti-MEM I to 1 μg/μl, and stored at -20°C until use.

Mice

ICR (CLEA Japan, Inc.) and B6D2F1 (C57BL/6 x DBA2 F1) (Japan SLC, Inc.) female mice were used. The ICR strain was mainly used for determining suitable conditions for electroporation, and the B6D2F1 strain was used for genome editing.

Embryo collection

Fertilized eggs were collected from the oviducts of E0.5 ICR or B6D2F1 females naturally intercrossed with males of the same strain. The figure following E corresponds to the number of days from the fertilization. E0.5 means 12 hours after the midpoint of the day of vaginal plug. The covering cumulus cells were removed by incubating in 1% hyaluronidase/M2 medium (Sigma). For the genome editing experiments targeting *H2b-mCherry,* the eggs were obtained from B6D2F1 females intercrossed with R26-H2b-mCherry males (RIKEN CDB, Japan) . The collected eggs were pre-cultured in mWM medium (ARK Resource, Japan) or KSOM medium (95 mM NaCl, 2.5 mM KCl, 0.35 mM $KH_2PO_4.7H_2O$, 0.2 mM $MgSO_4.7H_2O$, 0.2 mM glucose, 10 mM sodium lactate, 25 mM $NaHCO_3$, 0.2 mM sodium pyruvate, 1.71 mM $CaCl_2$-$2H_2O$, 0.01 mM $Na_2$-EDTA.$2H_2O$, 1 mM L-glutamine, 1 mg/ml BSA) until electroporation.

Electroporation

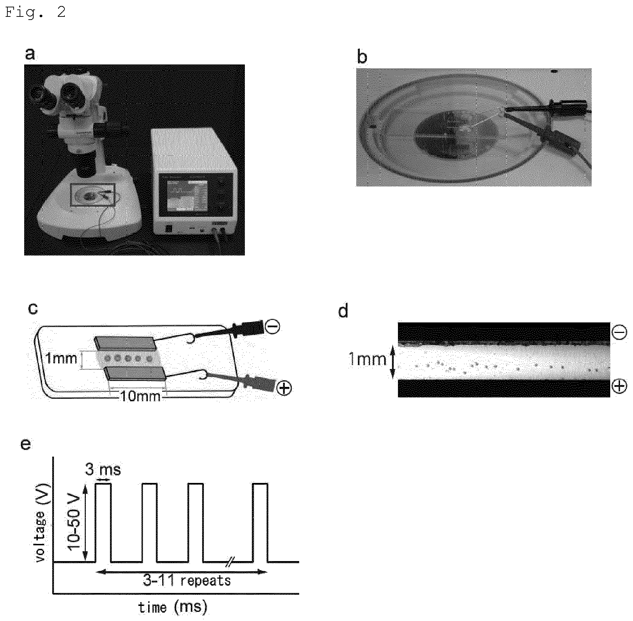

A pair of custom-made (BEX, Tokyo, Japan) platinum block electrodes (length: 10 mm, width: 3 mm, height: 0.5 mm, gap: 1 mm) was used (Fig. 2a). The electrodes, connected to a CUY21EDIT II (BEX) or CUY21 Vivo-SQ (BEX), were set under a stereoscopic microscope. The collected eggs cultured in mWM medium were washed with Opti-MEM I (Life technologies) three times to remove the serum-containing medium. The eggs were then placed in a line in the electrode gap filled with RNA-containing Opti-MEM I solution (total 5 μl volume), and electroporation was performed. The electroporation conditions were 30 V (3 msec pulse (ON) + 97 msec interval (OFF)) × 7 times unless otherwise stated. After electroporation, the eggs were immediately collected from the electrode gap and subjected to four washes with M2 medium followed by two washes with mWM medium. The eggs were then cultured in mWM medium at 37°C in a 5% $CO_2$ incubator until the two-cell stage.

Fluorescent signal detection and analyses

The signal intensity of the mCherry fluorescence was measured 15 hours after electroporation, using a Nipkow-disc confocal unit CSU-W1 (Yokogawa, Japan) connected to an Axio Observer Z1 inverted microscope (Zeiss, Germany). The fluorescent signal was detected by an EM-CCD camera ImageM (Hamamatsu Photonics, Japan) and the data were analyzed using the HC image software and NIH ImageJ (http://imagej.nih.gov/ij/). The signal intensity is obtained as a relative value depending on the conditions of the measurement and analysis, and can be compared only when all the conditions are same.

Genome editing of *Fgf10* and *H2b-mCherry*

The *Cas9* mRNA and gRNAs targeting *Fgf10* or *H2b-mCherry* were introduced into eggs collected from B6D2F1 females by electroporation at E0. 5 as described above. For the HDR-mediated knock-in study, ssODN was introduced together with the *Cas 9* mRNA and gRNA. The sequence of the ssODN was as follows: *H2b-mCherry* (5'-AGTTCATGCGCTTCAAGGTGCACATGGAGGGCTCCGTGAATTCATAACTTCGTATAGCATA CATTATACGAAGTTATCGAGGGCGAGGGCCGCCCCTACGAGGGCACCCAGACCGCC-3' (SEQ ID NO: 7) and 5'-CGTGAACGGCCACGAGTTCGAGATATCGAGGGCGAGGGCGAGGGCCGCCC-3' (SEQ ID NO: 8)). The surviving 2-cell-stage embryos were transferred to the oviducts of pseudopregnant females on the day of the vaginal plug. Alternatively, the embryos were cultured in vitro until the blastocyst stage (E4.5).

To investigate CRISPR/Cas9-mediated mutation in the *Fgf10* or *H2b-mCherry* gene, the genomes were prepared from the yolk sac of the embryos. The genomic regions flanking the gRNA target were amplified by PCR using specific primers: *FgflO* Fwd (5' -CAGCAGGTCTTACCCTTCCA-3' (SEQ ID NO: 9)) and *Fgf10* Rev (5'-TACAGGGGTTGGGGACATAA-3' (SEQ ID NO: 10)), *H2b-mCherry* Fwd (5'-GAGGGCACTAAGGCAGTCAC-3' (SEQ ID NO: 11)) and *H2b-mCherry* Rev (5'-CCCATGGTCTTCTTCTGCAT-3' (SEQ ID NO: 12)). The PCR amplicons of *Fgf10* or *H2b-mCherry* were cloned into pMD20 (Takara Bio Inc., Shiga, Japan) vector. Ten plasmids from each embryo were isolated, and the genomic region was sequenced. Sequencing was performed using the BigDye terminator Cycle Sequencing Kit ver. 3.1 and ABI 3500 Genetic Analyzer (Applied Biosystems, Foster City, CA, USA).

EXAMPLE 1

Electroporation conditions for introducing mRNA into a mouse fertilized egg

The conditions suitable for introducing mRNA into a fertilized mouse egg without treating the zona pellucida with an acid were studied. Electroporation set-up shown in Fig. 2a was used. The platinum block electrodes (gap: 1 mm, length: 10 mm, width: 3 mm, height: 0.5 mm) (Fig. 2c), which can hold 5 μl of a solution in the gap, were set under a stereoscopic microscope (Fig. 2a, left) and connected to an electroporator (CUY21EDIT II) (Fig. 2a, right). This system can treat about 40 to 50 eggs at once. Fertilized mouse eggs were manually positioned into a line prior to electroporation. *mCherry* mRNA (400 ng/μl) transcribed in vitro was used for electroporation and the efficiency of the mRNA introduction was evaluated by monitoring the fluorescence intensity of *mCherry* and the survival rate of the embryos at the blastocyst stage. Fig. 2a shows the electroporation set-up used in this study. Fig. 2b is higher magnification of the rectangle in Fig. 2a. Fig. 2c is a schematic drawing of the platinum block electrodes, showing that the eggs were placed in the RNA solution in the gap between the electrodes. Fig. 2d is a microscopic view of the eggs set in the electrode gap. Fig. 2e is a schematic drawing of the electroporation conditions used to introduce mRNAs into fertilized mouse eggs, showing that three to eleven repeats of a square pulse of 10-50V; 3-msec pulses with 97-msec intervals were used.

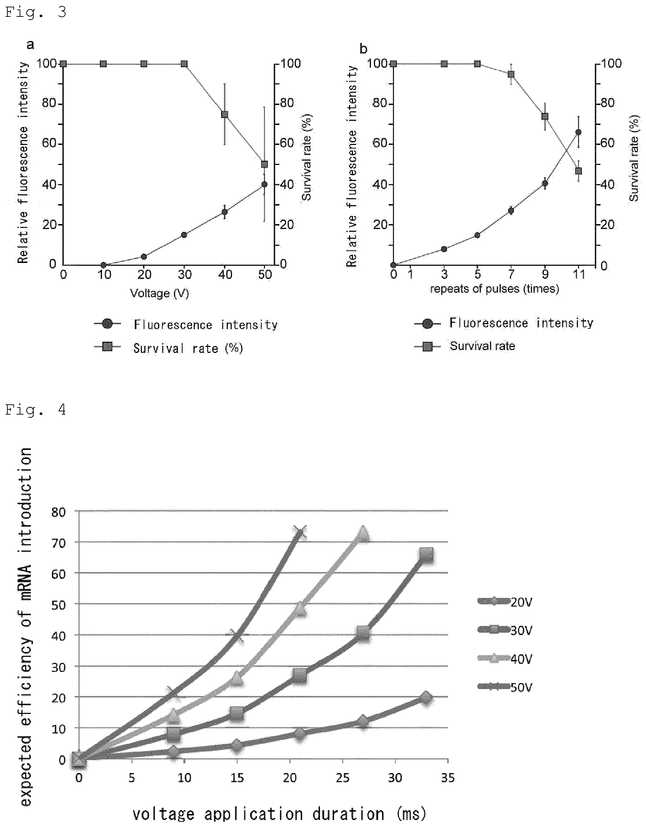

Fertilized eggs of E0.5 were electroporated at various voltages (10V-50V) while keeping the duration and number of the pulses fixed at 3 msec and five repeats, respectively. Fig. 3a shows the fluorescence intensity of *mCherry* (closed circles) and the survival rate of the electroporated embryos at the blastocyst stage (closed squares) plotted at the various voltages. The fluorescence was observed at the voltages of 20 V or more. The fluorescence intensity was 4.41 at the voltage of 20 V, 14.7 at the voltage of 30 V, 26.46 at the voltage of 40 V, and 39.69 at the voltage of 50 V, increasing with the voltages. Relative ratio of the fluorescence intensity at the voltage of 20 V, 30 V, 40 V, and 50 V was 0.3, 1.0, 1.8, and 2.7, respectively, when the fluorescence intensity at the voltage of 30 V was taken as 1.0. The survival rate was 100% at the voltages not more than 30 V, decreased along with the voltages at the voltage of 40 V or more, decreased to about 50% at the voltage of 50 V.

The voltage and duration of each pulse was fixed at 30 V and 3 msec, respectively, and the number of pulses was varied (x3, x5, x7, x9, and x11). Fig. 3b shows the fluorescence intensity of *mCherry* (closed circle) and the survival rate of the electroporated embryos at the blastocyst stage (closed squares) which were plotted as a function of the number of electroporation repeats. The fluorescence intensity increased with the number of repeats, being 7.9 at three repeats, 14.7 at five repeats, 27.1 at seven repeats, 40.6 at nine repeats, and 65.9 at eleven repeats. The survival rate of the electroporated embryos started to decrease at seven repeats and decreased to 50% at eleven repeats.

Since the fluorescence intensity of mCherry is proportional to the amount of introduced *mCherry* mRNA, the measured fluorescence intensity is a relative value of the efficiency of the mRNA introduction. Fig. 4 shows the expected efficiency of the mRNA introduction (R) for each voltage as a function of the voltage application duration. The expected efficiency was calculated on the basis of the fluorescence intensity shown in Fig. 3b and the relative ratio of the fluorescence intensity, which is 0.3, 1.0, 1.8, and 2.7 at the voltages of 20 V, 30 V, 40 V, and 50 V, respectively, derived from the data shown in Fig. 3a.

Mathematical functions that fit to the graph shown in Fig. 4 were constructed using Excel software. The efficiency of the mRNA introduction at each voltage may be calculated using the following functions, in which t is the voltage application duration (msec):

$$20\text{ V}: R = 0.0005 \times t^3 - 0.0057 \times t^2 + 0.2847 \times t;$$
$$30\text{ V}: R = 0.0015 \times t^3 - 0.0191 \times t^2 + 0.9489 \times t;$$
$$40\text{ V}: R = 0.0005 \times t^3 + 0.0508 \times t^2 + 0.9922 \times t;$$
$$50\text{ V}: R = 0.0078 \times t^3 - 0.1414 \times t^2 + 3.0103 \times t.$$

EXAMPLE 2

Electroporation by pulses of the both direction

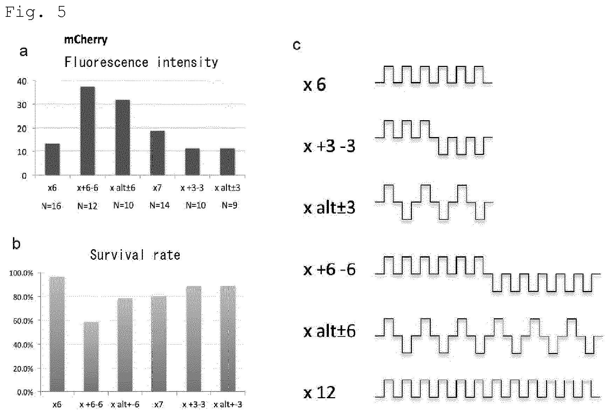

Similarly to EXAMPLE 1, *mCherry* mRNA was introduced to fertilized eggs of E0.5 by electroporation. The voltage and duration of each pulse were fixed at 30 V and 3 msec, respectively, and the number and direction of the pulses were changed as shown in Fig. 5c. In Fig. 5, "x6" indicates that six pulses of the same direction were applied, "x+3-3" indicates three pulses of one direction were sequentially applied and then three pulses of the opposite direction were applied, and "xalt±3" indicates three pulses of one direction and three pulses of the opposite direction were alternately applied. The same is applied to "x+6-6" and "xalt±6". The fluorescence intensity of mCherry increased with the number of the pulses irrespective of direction of the voltage (Fig. 5a). The survival rate of the electroporated embryos at the blastocyst stage was high in the all cases (Fig. 5b). Especially, "x+6-6" or "xalt±6" resulted in higher survival rate than "x12", which indicates 12 pulses of the same direction were applied.

EXAMPLE 3

Genome editing of *FgflO* gene by *Cas9* mRNA and gRNA introduced by electroporation

Whether the electroporation conditions above were conducive to CRISPR/Cas9-mediated genome editing was studied.

*FgflO* gene was targeted, because *FgflO* homozygous mutant embryos have a limbless phenotype, which enables easy detection of gene destruction (Sekine, K. et al., Nature Genetics 21, 138-141(1999)). Furthermore, it was previously confirmed that CRISPR/Cas system successfully destroyed the gene when *Cas9* mRNA and gRNA were microinjected (Yasue, A. et al., Scientific Reports 4, 5705 (2014)).

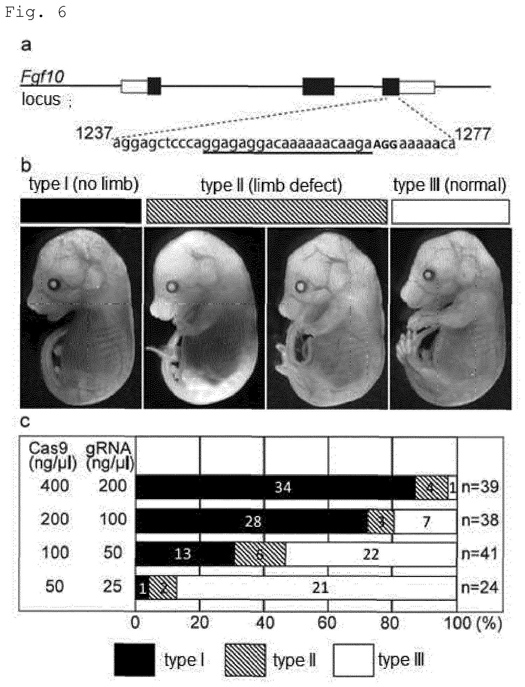

gRNA designated #563, which targets *FgflO* and comprises the nucleotide sequence of SEQ ID NO: 5, was used. The same gRNA was also used in Yasue, A. et al. Various concentrations of *Cas9* mRNA and the gRNA were introduced to fertilized eggs of E0.5 by electroporation, wherein seven pulses of 30 V and 3 msec were applied. Fig. 6a shows genomic structure of the *FgflO* locus, which includes the target sequence (underlined) and the PAM sequence (AGG, capitalized), used in this study. The eggs were allowed to develop to the two-cell stage and then transferred into pseudopregnant females. The mice were dissected at E15 or E16, and phenotype of the embryos was analyzed. Depending on the limb-development defects observed at E15 or E16, the embryos were classified into three categories of phenotype: type I embryos had no limbs (*Fgf10* gene knockout phenotype), type II embryos showed various defects in limb morphology (e.g., hindlimb deficiency or truncated fore- and hindlimbs), and type III embryos appeared normal. Fig. 6b shows representatives of the three categories, no limb, limb defects (left: hindlimb deficiency, right: truncated fore- and hind-limb), and normal. Fig. 6c shows a graph summarizing the effects of Cas9 and gRNA electroporation on limb development. The RNA concentrations used in each experiment are shown at left. The numbers in each row are the number of the embryos that exhibited the phenotype of each category.

Table 1 shows the concentration of the *Cas9* mRNA and gRNA used, the survival rate of the embryos at the two-cell stage, and the survival rate of the embryos at E15 or E16. Table 2 shows that the *Fgf10* mutant embryos were successfully generated by *Cas9* mRNA and gRNA electroporation.

Table 1: Survival rate of electroporated embryos

| Cas9 (ng/ μl) | gRNA (ng/ μl) | No. transferred embryos/ No. electroporated embryos (survival rate at two-cell stage, %) | No. embryos at E15 or E16/ No. transferred embryos (survival rate at E15 or E16, %) |
|---|---|---|---|
| 400 | 200 | 75/80 (94) | 39/75 (52) |
| 200 | 100 | 60/63 (95) | 38/60 (63) |
| 100 | 50 | 60/64 (94) | 43/60 (72) |
| 50 | 25 | 33/35 (94) | 17/33 (51) |

Table 2: Defects in limb morphology in electroporated embryos

| RNA conc. | | total No. embryos | No. embryos | | |
|---|---|---|---|---|---|
| Cas9 (ng/μl) | gRNA (ng/μl) | | Type I No limb | Type II Limb defect | Type III Normal |
| 400 | 200 | 39 | 34 | 4 | 1 |
| 200 | 100 | 38 | 28 | 3 | 7 |
| 100 | 50 | 41 | 13 | 6 | 22 |
| 50 | 25 | 24 | 1 | 2 | 21 |

The survival rate of the electroporated embryos that developed to the two-cell stage (94-95%; Table 1) was much higher than embryos subjected to the microinjection method (34-35% in Yasue, A. et al.).

When 400 ng/μl *Cas9* mRNA and 200 ng/μl gRNA were used for the electroporation, 97% (38/39) of the embryos displayed the characteristic limb defects. Among them 34 embryos completely lacked both fore- and hindlimbs, as expected from the *Fgf10* homozygous mutant phenotype obtained by conventional gene targeting. Four embryos displayed various other limb defects. When 200 ng/µl *Cas9* mRNA and 100 ng/µl gRNA were used for the electroporation, 82% (31/38) of the embryos displayed limb defects. When 100 ng/µl *Cas9* mRNA and 50 ng/µl gRNA were used, 46% (19/41) of the embryos displayed at least partial limb defects. When 50 ng/µl *Cas9* mRNA and 25 ng/µl gRNA were used, most of the embryos appeared normal.

To reveal whether the *Fgf10* gene was disrupted in the embryos, the genomic sequence of the embryo was analyzed. The genomic region flanking the target sequence was amplified and sequenced for ten clones each from four randomly selected embryos. The wild-type sequence of the genomic region flanking the target sequence is tgaatggaaaaggagctcccaggagaggacaaaaaacaagaAGGaaaaacacctctgctca (the target sequence is underlined. Capital letters indicate PAM sequence (AGG)) (SEQ ID NO: 13). The results are shown in Table 3.

Table 3: Sequence analysis of *Fgf10* mutants

| RNA conc. (ng/µl) Cas9/gRNA | Embryo ID | Type of mutation | No. clones |
|---|---|---|---|
| 400/200 | #1 | 15 bp deletion | 5 |
| | | 26 bp deletion | 3 |
| | | 3 bp deletion | 2 |
| | #2 | 13 bp deletion | 6 |
| | | 14 bp deletion | 4 |
| | #3 | 38 bp deletion | 4 |
| | | 6 bp deletion | 4 |
| | | 14 bp deletion | 1 |
| | | 1 bp insertion | 1 |
| | #4 | 10 bp deletion | 2 |
| | | 15 bp deletion | 2 |
| | | 14 bp deletion | 2 |
| | | 1 bp insertion | 2 |
| 200/100 | #12 | 13 bp deletion | 3 |
| | | 10 bp deletion | 3 |
| | | 13 bp deletion | 2 |
| | | 3 bp insertion | 1 |
| | | 1 bp insertion | 1 |
| | #13 | 7 bp deletion | 2 |
| | | 1 bp insertion | 2 |
| | | 1 bp insertion | 2 |
| | | 15 bp deletion | 1 |
| | | 1 bp deletion | 1 |
| | #14 | 15 bp deletion | 5 |
| | | 6 bp deletion | 2 |
| | | 15 bp deletion | 1 |
| | #15 | 1 bp insertion | 5 |
| | | 13 bp deletion | 3 |
| | | 47 bp or more deletion | 1 |

(continued)

| RNA conc. (ng/μl) Cas9/gRNA | Embryo ID | Type of mutation | No. clones |
|---|---|---|---|
| 100/50 | #16 | 13 bp deletion | 5 |
| | | 1 bp insertion | 5 |
| | #17 | 13 bp deletion | 8 |
| | | 3 bp deletion | 1 |
| | #18 | wild type | 8 |
| | | 15 bp deletion | 2 |
| | #19 | wild type | 8 |
| | | 1 bp mutation | 1 |
| 50/25 | #28 | wild type | 8 |
| | | 2 bp insertion | 1 |
| | | 1 bp mutation | 1 |
| | #29 | wild type | 7 |
| | | 3 bp deletion | 3 |
| | #30 | wild type | 5 |
| | | 1 bp mutation | 2 |
| | | 2 bp insertion | 2 |
| | | 2 bp deletion | 1 |
| | #31 | wild type | 8 |
| | | 1 bp mutation | 1 |
| | | 1 bp mutation | 1 |

In the table, when the same type of mutation is listed twice or more for one embryo, the sequences of each clone are different.

When 400 ng/μl *Cas9* mRNA and 200 ng/μl gRNA were used, all of the sequenced clones carried nucleotide insertion or deletion (indel) or mutation, and no wild-type sequence was detected. These results indicate that both alleles of the *Fgf10* gene were disrupted when 400 ng/μl *Cas9* mRNA and 200 ng/μl gRNA were used for the electroporation. Furthermore, each embryo had not more than four types of mutation, suggesting that the genome editing occurred immediately after the electroporation at the one-cell or two-cell stage. When 50 ng/μl *Cas 9* mRNA and 25 ng/μl gRNA were used, the most of the embryos appeared normal, but sequencing revealed that some clones derived from the embryos carried an indel or mutation.

The results indicate that electroporation can be used for CRISPR/Cas-mediated genome editing and the efficiency of the genome editing depends on the RNA concentration. The high concentration of *Cas9* mRNA and gRNA disrupted both alleles in the almost all cells, whereas the low concentration gave chimeric embryos comprising mutant cells, in which either or both of the alleles are mutated, and wild-type cells.

EXAMPLE 4

Genome editing of *mCherry* gene by *Cas 9* mRNA and gRNA introduced by electroporation

Fertilized eggs carrying a *Histone H2b (H2b)-mCherry* gene inserted into the *ROSA26* locus were used (Abe et al., Genesis 49, 579-590 (2011)). The embryos developed from the eggs ubiquitously express *H2b-mCherry* under the control of the *Rosa26* promoter, exhibiting the mCherry fluorescence in the nucleus of the all cells at the four to eight-cell stages. When genome editing targeting *H2b-mCherry* occurs and the gene is disrupted, the *mCherry* fluorescence disappears.

In this study the lowest RNA concentration required for genome editing was determined when the voltage, duration and number of pulses of electroporation was fixed at 30 V, 3 msec and seven repeats, respectively. *Cas9* mRNA and gRNA targeting *H2b-mCherry* were introduced to the fertilized eggs of E0.5. The embryos were cultured in KSOM medium to the blastocyst stage (E4.5) and the *mCherry* fluorescence in the nuclei was detected. When 200 ng/μl or more of *Cas9* mRNA and 100 ng/μl or more of *mCherry* gRNA were used, no *mCherry* fluorescence was detected. When 50 to 100 ng/μl *Cas9* mRNA and 25 to 50 ng/μl gRNA were used, some blastomeres were mCherry-negative and others were positive. Electroporation using 25 ng/μl *Cas9* mRNA and 12.5 ng/μl gRNA had no effect on the *H2b-mCherry* expression, suggesting that the concentrations were too low to cause the genome editing under the fixed electroporation conditions employed herein.

Further experiments were for determining which concentration of *Cas9* mRNA and gRNA was important for the success of genome editing. When the concentration of *Cas9* mRNA was fixed at 25 ng/μl and the concentration of gRNA was varied, genome editing did not occur even if gRNA concentration as high as 200 ng/μl was used. On the other hand, when 200 ng/μl *Cas9* mRNA was used, genome editing did occur even if gRNA was decreased to 10 ng/μl. The same result was obtained when gRNA targeting the *FgflO* gene was used. The results suggest that the success of genome editing depends not on the concentration of gRNA, but on the concentration of Cas9 mRNA.

EXAMPLE 5

Genome editing mediated by high concentration of *Cas9* mRNA introduced by electroporation

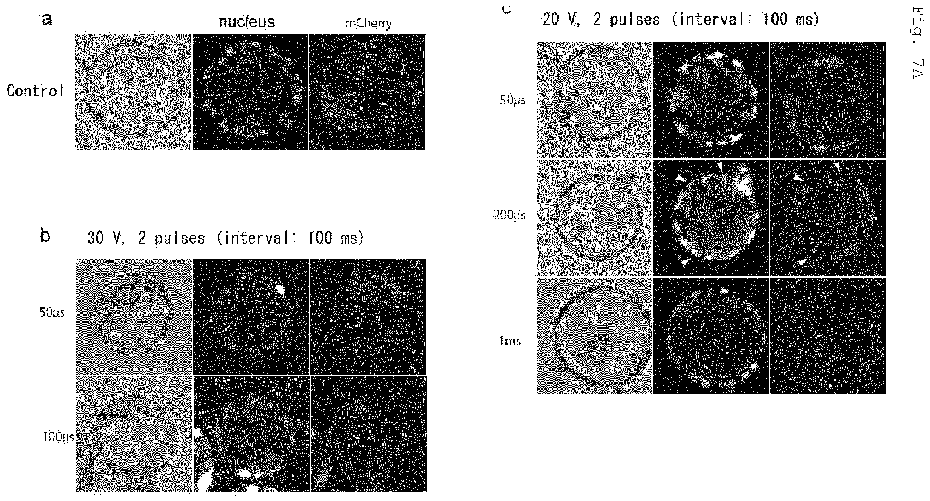

Similarly to EXAMPLE 4, electric conditions required for achieving genome editing when 2000 ng/μl *Cas9* mRNA and 1000 ng/μl gRNA were used was determined. *Cas9* mRNA and gRNA was introduced to fertilized mouse eggs of E0.5. The embryos were cultured in vitro to the blastocyst stage (E4.5) and the *mCherry* fluorescence in the nuclei was detected. The results are shown in Fig. 7. The *mCherry* fluorescence was detected in all blastomeres when electroporation was not performed (Fig. 7a). When electroporation was performed by applying pulses of 30 V, 0.05 msec and two repeats, the *mCherry* fluorescence was not detected in some blastomeres (Fig. 7b). When electroporation was performed by applying pulses of 30 V, 0.10 msec and two repeats, the fluorescence was not detected in any blastomere (Fig. 7b). When electroporation was performed by applying pulses of 20 V, 0.05 msec and two repeats, the fluorescence was detected in all blastomeres (Fig. 7c). When electroporation was performed by applying pulses of 20 V, 0.20 msec and two repeats, the fluorescence was not detected in some blastomeres (Fig. 7c, arrow heads). When electroporation was performed by applying pulses of 20 V, 1.00 msec and two repeats, the fluorescence was not detected in any blastomere (Fig. 7c).

EXAMPLE 6

Electroporation condition for achieving genome editing

Similarly to EXAMPLE 4, electric conditions required for achieving genome editing when 200 ng/μl *Cas9* mRNA and 100 ng/μl gRNA were used was determined. *Cas9* mRNA and gRNA was introduced to 5 to 12 fertilized eggs of E0.5 at the same time by electroporation using various voltages (20 to 50 V) and durations of pulses (6 to 33 msec). When electroporation conditions shown in Table 4 were employed, the mCherry fluorescence was not detected in some blastomeres of the embryos of E4.5, suggesting that genome editing was achieved.

Table 4: Electroporation conditions under which genome editing was achieved

| Voltage (V) | Duration (msec) |
|---|---|
| 20 | 15 |
| 20 | 18 |
| 20 | 30 |
| 20 | 33 |
| 30 | 9 |
| 30 | 12 |
| 30 | 15 |
| 30 | 21 |
| 30 | 24 |

(continued)

| Voltage (V) | Duration (msec) |
|---|---|
| 50 | 6 |
| 50 | 9 |

When 200 ng/ul *Cas9* mRNA was used, genome editing was achieved by applying voltage of 20 V for 15 msec. As measured in EXAMPLE 1, the efficiency of mRNA introduction under this electric condition is 4.41.

EXAMPLE 7

Introduction of ssODN by electroporation to lead homology directed repair

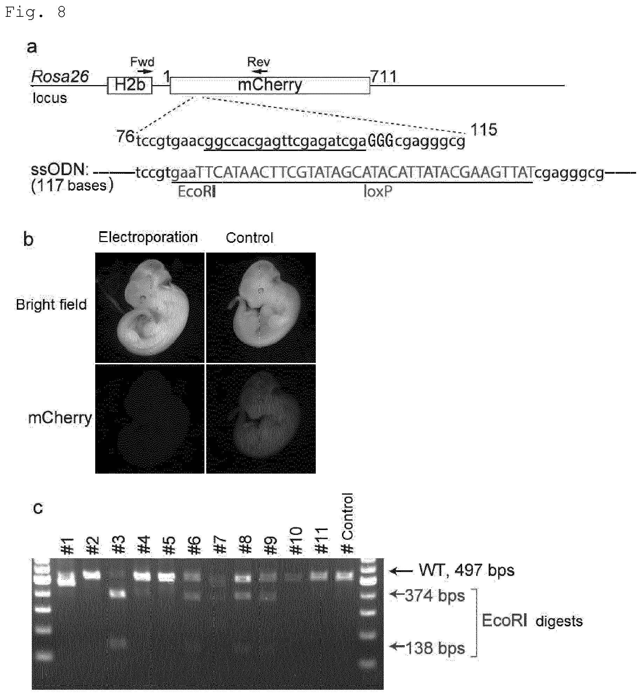

Whether electroporation could deliver ssODNs to a fertilized mouse egg and generate HDR-mediated knock-in alleles was examined. ssODN of 117 bases harboring loxP and EcoRI recognition sequences (37 bases) flanked by 40-base homologous arms was used (SEQ ID NO: 7) . *Cas9* mRNA, gRNA targeting the *mCherry* gene and the ssODN were introduced into the fertilized eggs that carry a *Histone H2b (H2b)-mCherry* gene inserted into the *ROSA26* locus (see EXAMPLE 4) by electroporation. The embryos were cultured to the two-cell stage and transferred to pseudopregnant females. Fig. 8a shows a schematic drawing of the target sequence and the ssODN designed to insert the 37-base loxP sequence and EcoRI recognition site. The allele replaced by the ssODN would be functionally null due to the introduction of a stop codon in the loxP sequence, causing the disappearance of the nuclear *mCherry* fluorescence. Replacement by the ssODN was screened by Restriction Fragment Length Polymorphism (RFLP) analysis after EcoRI digestion.

All of the electroporated embryos (11/11) exhibited a loss of mCherry fluorescence. Fig. 8b shows representative images of the embryo subjected to the electroporation. The mCherry fluorescence completely disappeared in the electroporated embryo, while the control embryo, which was not subjected to the electroporation, displayed the fluorescent signals. Fig. 8c shows the results of the RFLP analysis of the collected embryos. The EcoRI-inserted alleles were digested into two bands (138 bps and 374 bps). The intact allele had 497 bps. The digested bands were observed in embryos #3, #6, #8, and #9, indicating that the four embryos were positive for EcoRI digestion among the embryos that lost the fluorescent. The unexpected bands in #1 and #7 indicate that a large deletion was generated in the target gene.

Table 5 shows the results of the sequence analysis of the embryos positive for EcoRI digestion. The wild-type sequence of the genomic regions flanking the target sequence is gtgaac<u>ggccacgagttcgagatcga</u>GGGcga (the target sequence is underlined. Capital letters indicate PAM sequence (GGG)) (SEQ ID NO: 14).

Table 5: Sequence analysis of embryos subjected to HDR-mediated knock-in of loxP and EcoRI site

| Embryo ID | Type of mutation | No. clones |
|---|---|---|
| #3 | HDR-mediated knock-in | 10/10 |
| #6 | HDR-mediated knock-in | 3/9 |
| | 1 bp insertion | 3/9 |
| | unexpected insertion of ssODN | 2/9 |
| | 1 bp deletion | 1/9 |
| #8 | HDR-mediated knock-in | 5/8 |
| | 1 bp deletion | 2/8 |
| | unexpected insertion of ssODN | 1/8 |
| #9 | HDR-mediated knock-in | 6/8 |
| | 1 bp deletion | 2/8 |

Sequencing revealed that all the four embryos carried the HDR-mediated replaced allele. Among them, three embryos (#6, #8, and #9) carried one to three types of alleles with indels, in addition to the replaced allele. The remaining embryo (#3) carried only the replaced allele, indicating that all of the cells carried an allele with the HDR-mediated replacement sequence.

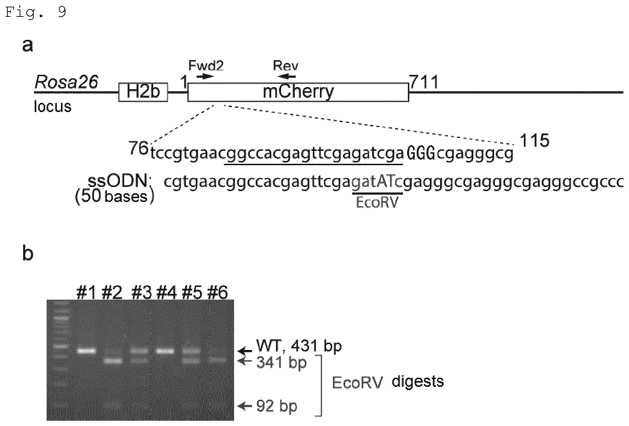

In further experiments, HDR-mediated knock-in of an EcoRV site into the *mCherry* gene was also achieved (Table 6 and Fig. 9) . Fig. 9a shows a schematic drawing of the target sequence and the ssODN designed to insert the EcoRV recognition site (SEQ ID NO: 8). Fig. 9b shows the results of the RFLP analysis of the collected embryos. The EcoRV-inserted alleles were digested into two bands (341 bps and 92 bps). The intact allele had 431 bps. The digested bands were observed in embryos #2, #3, #5, and #6.

Table 6 shows the results of the sequence analysis of the embryos. The wild-type sequence of the genomic regions flanking the target sequence is gtgaacggccacgagttcgagatcgaGGGcga (the target sequence is underlined. Capital letters indicate PAM sequence (GGG)) (SEQ ID NO: 14).

Table 6: Sequence analysis of embryos subjected to HDR-mediated knock-in of EcoRV site

| Embryo ID | Type of mutation | No. clones |
|---|---|---|
| #1 | 1 bp insertion | 4/10 |
| | 1 bp insertion | 3/10 |
| | 1 bp insertion | 3/10 |
| #2 | HDR-mediated knock-in | 10/10 |
| #3 | 1 bp deletion | 5/9 |
| | HDR-mediated knock-in | 3/9 |
| | 4 bp deletion | 1/9 |
| #4 | 1 bp deletion | 5/10 |
| | 1 bp insertion | 1/10 |
| | unexpected insertion of ssODN | 3/10 |
| #5 | 4 bp deletion | 3/10 |
| | HDR-mediated knock-in | 6/10 |
| | wild type | 1/10 |
| #6 | HDR-mediated knock-in | 1/9 |
| | unexpected insertion of ssODN | 1/9 |
| | unexpected insertion of ssODN | 7/9 |

In the table, when the same type of mutation is listed twice or more for one embryo, the sequences of each clone are different.

In further experiments, the HDR-mediated knock-in of an XbaI site into the *Fgf10* gene was also achieved (data not shown). The results indicate that not only *Cas9* mRNA and gRNA but also ssODN can be introduced to embryos by electroporation and HDR-mediated knock-in alleles are generated.

## Claims

**1.** A method of introducing mRNA encoding Cas9 protein (*Cas9* mRNA) into a non-human mammalian embryo, comprising the steps of;

(a) placing a mixture of the non-human mammalian embryo and a solution comprising *Cas9* mRNA in the gap between a pair of electrodes, wherein the concentration of *Cas9* mRNA is at least 200 ng/μl, and
(b) applying a voltage to the electrodes for a voltage application duration,

wherein the voltage is at least 20 V per millimeter of the distance between the electrodes, and the voltage application duration is at least 15 msec; or
wherein the voltage is at least 30 V per millimeter of the distance between the electrodes, and the voltage application duration is at least 5 msec,
provided that the voltage is 20 to 40 V per millimeter of the distance between the electrodes, and the product of the voltage and the voltage application duration is not more than 990 Vmsec per millimeter of the distance between the electrodes.

**2.** The method according to claim 1, wherein the voltage is 25 to 35 V per millimeter, preferably 30 V, of the distance between the electrodes.

**3.** The method according to claim 1 or 2, wherein the voltage is at least 20 V per millimeter of the distance between the electrodes, and the voltage application duration is at least 15 msec.

**4.** The method according to claim 1 or 2, wherein the voltage is at least 30 V per millimeter of the distance between the electrodes, and the voltage application duration is at least 5 msec.

**5.** The method according to any one of claims 1 to 4, wherein the embryo is a rodent embryo, preferably a mouse embryo.

**6.** The method according to any one of claims 1 to 5, wherein the Cas9 protein comprises an amino acid sequence having at least 90% amino acid sequence identity with the amino acid sequence of any one of SEQ ID NOs: 1 to 4, preferably wherein the CAS9 protein comprises the amino acid sequence of any one of SEQ ID NOs: 1 to 4, most preferably the amino acid sequence of SEQ ID NO: 1, and has an ability to bind to DNA in the presence of gRNA, preferably further has RuvC and/or HNH nuclease activity.

**7.** The method according to any one of claims 1 to 6, wherein the solution comprises at least one further nucleic acid, the nucleic acid is gRNA or combination of crRNA and tracrRNA, preferably gRNA, and the nucleic acid is introduced to the embryo together with the *Cas9* mRNA.

**8.** The method according to claim 7, wherein the solution further comprises single-stranded oligodeoxynucleotide (ssODN).

**9.** A method of preparing a non-human mammalian embryo expressing Cas9 protein, comprising introducing *Cas9* mRNA into a non-human mammalian embryo by the method according to any one of claims 1 to 8.

**10.** A method of performing genome editing in a non-human mammalian embryo, comprising introducing Cas9 mRNA and a further nucleic acid into the non-human mammalian embryo by the method according to claim 7 or 8.

11. A method of preparing a non-human mammalian embryo whose genome is modified by genome editing, comprising introducing *Cas9* mRNA and a further nucleic acid into a non-human mammalian embryo by the method according to claim 7 or 8.

12. A method of preparing a genetically modified non-human animal, comprising transferring the embryo obtained by the method according to claim 11 to a non-human recipient animal.

**Patentansprüche**

1. Verfahren zum Einführen von mRNA, die das Cas9-Protein codiert (Cas9-mRNA), in einen nichthumanen Säugerembryo, umfassend die Schritte:

   (a) Platzieren eines Gemischs des nichthumanen Säugerembryos und einer Lösung, die Cas9-mRNA umfasst, in der Lücke zwischen einem Paar von Elektroden, wobei die Konzentration von Cas9-mRNA wenigstens 200 ng/μl beträgt; und
   (b) Anlegen einer Spannung an die Elektroden während einer Spannungsanlegungsdauer;

   wobei die Spannung wenigstens 20 V pro Millimeter Abstand zwischen den Elektroden beträgt und die Spannungsanlegungsdauer wenigstens 15 ms beträgt; oder
   wobei die Spannung wenigstens 30 V pro Millimeter Abstand zwischen den Elektroden beträgt und die Spannungsanlegungsdauer wenigstens 5 ms beträgt;
   mit der Maßgabe, dass die Spannung 20 bis 40 V pro Millimeter Abstand zwischen den Elektroden beträgt und das Produkt der Spannung und der Spannungsanlegungsdauer nicht mehr als 990 Vms pro Millimeter Abstand zwischen den Elektroden beträgt.

2. Verfahren gemäß Anspruch 1, wobei die Spannung 25 bis 35 V, vorzugsweise 30 V, pro Millimeter Abstand zwischen den Elektroden beträgt.

3. Verfahren gemäß Anspruch 1 oder 2, wobei die Spannung wenigstens 20 V pro Millimeter Abstand zwischen den Elektroden beträgt und die Spannungsanlegungsdauer wenigstens 15 ms beträgt.

4. Verfahren gemäß Anspruch 1 oder 2, wobei die Spannung wenigstens 30 V pro Millimeter Abstand zwischen den Elektroden beträgt und die Spannungsanlegungsdauer wenigstens 5 ms beträgt.

5. Verfahren gemäß einem der Ansprüche 1 bis 4, wobei der Embryo ein Nagerembryo, vorzugsweise ein Mausembryo, ist.

6. Verfahren gemäß einem der Ansprüche 1 bis 5, wobei das Cas9-Protein eine Aminosäuresequenz umfasst, die wenigstens 90% Aminosäuresequenzidentität mit der Aminosäuresequenz von einem von SEQ ID Nr. 1 bis 4 aufweist, wobei vorzugsweise das Cas9-Protein die Aminosäuresequenz von einem von SEQ ID Nr. 1 bis 4, am meisten bevorzugt die Aminosäuresequenz von SEQ ID Nr. 1, umfasst und die Fähigkeit aufweist, in Gegenwart von gRNA an DNA zu binden, vorzugsweise weiterhin RuvC- und/oder HNH-Nuclease-Aktivität aufweist.

7. Verfahren gemäß einem der Ansprüche 1 bis 6, wobei die Lösung wenigstens eine weitere Nucleinsäure umfasst, es sich bei der Nucleinsäure um gRNA oder eine Kombination von crRNA und tracrRNA, vorzugsweise gRNA, handelt und die Nucleinsäure zusammen mit der Cas9-mRNA in den Embryo eingeführt wird.

8. Verfahren gemäß Anspruch 7, wobei die Lösung weiterhin einzelsträngiges Oligodesoxynucleotid (ssODN) umfasst.

9. Verfahren zur Herstellung eines nichthumanen Säugerembryos, der Cas9-Protein exprimiert, umfassend das Einführen von Cas9-mRNA in einen nichthumanen Säugerembryo nach dem Verfahren gemäß einem der Ansprüche 1 bis 8.

10. Verfahren zum Durchführen von Genom-Editing in einem nichthumanen Säugerembryo, umfassend das Einführen von Cas9-mRNA und einer weiteren Nucleinsäure in den nichthumanen Säugerembryo nach dem Verfahren gemäß Anspruch 7 oder 8.

11. Verfahren zur Herstellung eines nichthumanen Säugerembryos, dessen Genom durch Genom-Editing modifiziert ist, umfassend das Einführen von Cas9-mRNA und einer weiteren Nucleinsäure in einen nichthumanen Säugerembryo nach dem Verfahren gemäß Anspruch 7 oder 8.

12. Verfahren zur Herstellung eines genetisch modifizierten nichthumanen Tiers, umfassend das Übertragen des nach dem Verfahren gemäß Anspruch 11 erhaltenen Embryos auf ein nichthumanes Empfängertier.

**Revendications**

1. Procédé d'introduction d'ARNm codant la protéine Cas9 (ARNm de *Cas9*) dans un embryon de mammifère non humain, comprenant les étapes de;

   (a) placer un mélange de l'embryon de mammifère non humain et d'une solution comprenant de l'ARNm de *Cas9* dans l'espace entre une paire d'électrodes, où la concentration d'ARNm de *Cas9* est d'au moins 200 ng/μl, et
   (b) appliquer une tension aux électrodes pendant une durée d'application de la tension,

      où la tension est d'au moins 20 V par millimètre de la distance entre les électrodes, et la durée d'application de la tension est d'au moins 15 ms; ou
      où la tension est d'au moins 30 V par millimètre de la distance entre les électrodes, et la durée d'application de la tension est d'au moins 5 ms,
      à condition que la tension soit de 20 à 40 V par millimètre de la distance entre les électrodes, et que le produit de la tension et de la durée d'application de la tension ne soit pas supérieur à 990 Vms par millimètre de la distance entre les électrodes.

2. Procédé selon la revendication 1, où la tension est de 25 à 35 V par millimètre, de préférence 30 V, de la distance entre les électrodes.

3. Procédé selon la revendication 1 ou 2, où la tension est d'au moins 20 V par millimètre de la distance entre les électrodes, et la durée d'application de la tension est d'au moins 15 ms.

4. Procédé selon la revendication 1 ou 2, où la tension est d'au moins 30 V par millimètre de la distance entre les électrodes, et la durée d'application de la tension est d'au moins 5 ms.

5. Procédé selon l'une quelconque des revendications 1 à 4, où l'embryon est un embryon de rongeur, de préférence un embryon de souris.

6. Procédé selon l'une quelconque des revendications 1 à 5, où la protéine Cas9 comprend une séquence d'acides aminés ayant au moins 90 % d'identité de séquence d'acides aminés avec la séquence d'acides aminés de l'une quelconque de SEQ ID NO: 1 à 4, de préférence où la protéine CAS9 comprend la séquence d'acides aminés de l'une quelconque de SEQ ID NO: 1 à 4, de manière particulièrement préférable la séquence d'acides aminés de SEQ ID NO: 1, et a une aptitude à se lier à l'ADN en présence d'ARNg, de préférence a en outre une activité de nucléase RuvC et/ou HNH.

7. Procédé selon l'une quelconque des revendications 1 à 6, où la solution comprend au moins un autre acide nucléique, l'acide nucléique est un ARNg ou une combinaison d'ARNcr et d'ARNtracr, de préférence un ARNg, et l'acide nucléique est introduit dans l'embryon conjointement avec l'ARNm de *Cas9.*

8. Procédé selon la revendication 7, où la solution comprend en outre un oligodésoxynucléotide simple brin (ODNsb).

9. Procédé de préparation d'un embryon de mammifère non humain exprimant la protéine Cas9, comprenant l'introduction d'ARNm de *Cas9* dans un embryon de mammifère non humain par le procédé selon l'une quelconque des revendications 1 à 8.

10. Procédé de réalisation d'édition du génome dans un embryon de mammifère non humain, comprenant l'introduction d'ARNm de *Cas9* et d'un autre acide nucléique dans l'embryon de mammifère non humain par le procédé selon la revendication 7 ou 8.

**11.** Procédé de préparation d'un embryon de mammifère non humain dont le génome est modifié par édition du génome, comprenant l'introduction d'ARNm de *Cas9* et d'un autre acide nucléique dans un embryon de mammifère non humain par le procédé selon la revendication 7 ou 8.

**12.** Procédé de préparation d'un animal non humain génétiquement modifié, comprenant le transfert de l'embryon obtenu par le procédé selon la revendication 11 à un animal receveur non humain.

Fig. 1-1

SEQ ID NO: 1
Amino acid sequence of Cas9 protein
derived from *Streptococcus pyogenes*

```
   1 MDKKYSIGLD IGTNSVGWAV ITDEYKVPSK KFKVLGNTDR HSIKKNLIGA LLFDSGETAE
  61 ATRLKRTARR RYTRRKNRIC YLQEIFSNEM AKVDDSFFHR LEESFLVEED KKHERHPIFG
 121 NIVDEVAYHE KYPTIYHLRK KLVDSTDKAD LRLIYLALAH MIKFRGHFLI EGDLNPDNSD
 181 VDKLFIQLVQ TYNQLFEENP INASGVDAKA ILSARLSKSR RLENLIAQLP GEKKNGLFGN
 241 LIALSLGLTP NFKSNFDLAE DAKLQLSKDT YDDDLDNLLA QIGDQYADLF LAAKNLSDAI
 301 LLSDILRVNT EITKAPLSAS MIKRYDEHHQ DLTLLKALVR QQLPEKYKEI FFDQSKNGYA
 361 GYIDGGASQE EFYKFIKPIL EKMDGTEELL VKLNREDLLR KQRTFDNGSI PHQIHLGELH
 421 AILRRQEDFY PFLKDNREKI EKILTFRIPY YVGPLARGNS RFAWMTRKSE ETITPWNFEE
 481 VVDKGASAQS FIERMTNFDK NLPNEKVLPK HSLLYEYFTV YNELTKVKYV TEGMRKPAFL
 541 SGEQKKAIVD LLFKTNRKVT VKQLKEDYFK KIECFDSVEI SGVEDRFNAS LGTYHDLLKI
 601 IKDKDFLDNE ENEDILEDIV LTLTLFEDRE MIEERLKTYA HLFDDKVMKQ LKRRRYTGWG
 661 RLSRKLINGI RDKQSGKTIL DFLKSDGFAN RNFMQLIHDD SLTFKEDIQK AQVSGQGDSL
 721 HEHIANLAGS PAIKKGILQT VKVVDELVKV MGRHKPENIV IEMARENQTT QKGQKNSRER
 781 MKRIEEGIKE LGSQILKEHP VENTQLQNEK LYLYYLQNGR DMYVDQELDI NRLSDYDVDH
 841 IVPQSFLKDD SIDNKVLTRS DKNRGKSDNV PSEEVVKKMK NYWRQLLNAK LITQRKFDNL
 901 TKAERGGLSE LDKAGFIKRQ LVETRQITKH VAQILDSRMN TKYDENDKLI REVKVITLKS
 961 KLVSDFRKDF QFYKVREINN YHHAHDAYLN AVVGTALIKK YPKLESEFVY GDYKVYDVRK
1021 MIAKSEQEIG KATAKYFFYS NIMNFFKTEI TLANGEIRKR PLIETNGETG EIVWDKGRDF
1081 ATVRKVLSMP QVNIVKKTEV QTGGFSKESI LPKRNSDKLI ARKKDWDPKK YGGFDSPTVA
1141 YSVLVVAKVE KGKSKKLKSV KELLGITIME RSSFEKNPID FLEAKGYKEV KKDLIIKLPK
1201 YSLFELENGR KRMLASAGEL QKGNELALPS KYVNFLYLAS HYEKLKGSPE DNEQKQLFVE
1261 QHKHYLDEII EQISEFSKRV ILADANLDKV LSAYNKHRDK PIREQAENII HLFTLTNLGA
1321 PAAFKYFDTT IDRKRYTSTK EVLDATLIHQ SITGLYETRI DLSQLGGD
```

Fig. 1-2

SEQ ID NO: 2
Amino acid sequence of Cas9 protein
derived from *Neisseria meningitides*

```
   1 MAAFKPNSIN YILGLDIGIA SVGWAMVEID EEENPIRLID LGVRVFERAE VPKTGDSLAM
  61 ARRLARSVRR LTRRRAHRLL RTRRLLKREG VLQAANFDEN GLIKSLPNTP WQLRAAALDR
 121 KLTPLEWSAV LLHLIKHRGY LSQRKNEGET ADKELGALLK GVAGNAHALQ TGDFRTPAEL
 181 ALNKFEKESG HIRNQRSDYS HTFSRKDLQA ELILLFEKQK EFGNPHVSGG LKEGIETLLM
 241 TQRPALSGDA VQKMLGHCTF EPAEPKAAKN TYTAERFIWL TKLNNLRILE QGSERPLTDT
 301 ERATLMDEPY RKSKLTYAQA RKLLGLEDTA FFKGLRYGKD NAEASTLMEM KAYHAISRAL
 361 EKEGLKDKKS PLNLSPELQD EIGTAFSLFK TDEDITGRLK DRIQPEILEA LLKHISFDKF
 421 VQISLKALRR IVPLMEQGKR YDEACAEIYG DHYGKKNTEE KIYLPPIPAD EIRNPVVLRA
 481 LSQARKVING VVRRYGSPAR IHIETAREVG KSFKDRKEIE KRQEENRKDR EKAAAKFREY
 541 FPNFVGEPKS KDILKLRLYE QQHGKCLYSG KEINLGRLNE KGYVEIDHAL PFSRTWDDSF
 601 NNKVLVLGSE NQNKGNQTPY EYFNGKDNSR EWQEFKARVE TSRFPRSKKQ RILLQKFDED
 661 GFKERNLNDT RYVNRFLCQF VADRMRLTGK GKKRVFASNG QITNLLRGFW GLRKVRAEND
 721 RHHALDAVVV ACSTVAMQQK ITRFVRYKEM NAFDGKTIDK ETGEVLHQKT HFPQPWEFFA
 781 QEVMIRVFGK PDGKPEFEEA DTLEKLRTLL AEKLSSRPEA VHEYVTPLFV SRAPNRKMSG
 841 QGHMETVKSA KRLDEGVSVL RVPLTQLKLK DLEKMVNRER EPKLYEALKA RLEAHKDDPA
 901 KAFAEPFYKY DKAGNRTQQV KAVRVEQVQK TGVWVRNHNG IADNATMVRV DVFEKGDKYY
 961 LVPIYSWQVA KGILPDRAVV QGKDEEDWQL IDDSFNFKFS LHPNDLVEVI TKKARMFGYF
1021 ASCHRGTGNI NIRIHDLDHK IGKNGILEGI GVKTALSFQK YQIDELGKEI RPCRLKKRPP
1081 VR
```

Fig. 1-3

SEQ ID NO: 3
Amino acid sequence of Cas9 protein derived from *Streptococcus thermophiles*

```
   1 MTKPYSIGLD IGTNSVGWAV TTDNYKVPSK KMKVLGNTSK KYIKKNLLGV LLFDSGITAE
  61 GRRLKRTARR RYTRRRNRIL YLQEIFSTEM ATLDDAFFQR LDDSFLVPDD KRDSKYPIFG
 121 NLVEEKAYHD EFPTIYHLRK YLADSTKKAD LRLVYLALAH MIKYRGHFLI EGEFNSKNND
 181 IQKNFQDFLD TYNAIFESDL SLENSKQLEE IVKDKISKLE KKDRILKLFP GEKNSGIFSE
 241 FLKLIVGNQA DFRKCFNLDE KASLHFSKES YDEDLETLLG YIGDDYSDVF LKAKKLYDAI
 301 LLSGFLTVTD NETEAPLSSA MIKRYNEHKE DLALLKEYIR NISLKTYNEV FKDDTKNGYA
 361 GYIDGKTNQE DFYVYLKKLL AEFEGADYFL EKIDREDFLR KQRTFDNGSI PYQIHLQEMR
 421 AILDKQAKFY PFLAKNKERI EKILTFRIPY YVGPLARGNS DFAWSIRKRN EKITPWNFED
 481 VIDKESSAEA FINRMTSFDL YLPEEKVLPK HSLLYETFNV YNELTKVRFI AESMRDYQFL
 541 DSKQKKDIVR LYFKDKRKVT DKDIIEYLHA IYGYDGIELK GIEKQFNSSL STYHDLLNII
 601 NDKEFLDDSS NEAIIEEIIH TLTIFEDREM IKQRLSKFEN IFDKSVLKKL SRRHYTGWGK
 661 LSAKLINGIR DEKSGNTILD YLIDDGISNR NFMQLIHDDA LSFKKKIQKA QIIGDEDKGN
 721 IKEVVKSLPG SPAIKKGILQ SIKIVDELVK VMGGRKPESI VVEMARENQY TNQGKSNSQQ
 781 RLKRLEKSLK ELGSKILKEN IPAKLSKIDN NALQNDRLYL YYLQNGKDMY TGDDLDIDRL
 841 SNYDIDHIIP QAFLKDNSID NKVLVSSASN RGKSDDVPSL EVVKKRKTFW YQLLKSKLIS
 901 QRKFDNLTKA ERGGLSPEDK AGFIQRQLVE TRQITKHVAR LLDEKFNNKK DENNRAVRTV
 961 KIITLKSTLV SQFRKDFELY KVREINDFHH AHDAYLNAVV ASALLKKYPK LEPEFVYGDY
1021 PKYNSFRERK SATEKVYFYS NIMNIFKKSI SLADGRVIER PLIEVNEETG ESVWNKESDL
1081 ATVRRVLSYP QVNVVKKVEE QNHGLDRGKP KGLFNANLSS KPKPNSNENL VGAKEYLDPK
1141 KYGGYAGISN SFTVLVKGTI EKGAKKKITN VLEFQGISIL DRINYRKDKL NFLLEKGYKD
1201 IELIIELPKY SLFELSDGSR RMLASILSTN NKRGEIHKGN QIFLSQKFVK LLYHAKRISN
1261 TINENHRKYV ENHKKEFEEL FYYILEFNEN YVGAKKNGKL LNSAFQSWQN HSIDELCSSF
1321 IGPTGSERKG LFELTSRGSA ADFEFLGVKI PRYRDYTPSS LLKDATLIHQ SVTGLYETRI
1381 DLAKLGEG
```

Fig. 1-4

SEQ ID NO: 4
Amino acid sequence of Cas9 protein derived from *Treponema denticola*

1 MKKEIKDYFL GLDVGTGSVG WAVTDTDYKL LKANRKDLWG MRCFETAETA EVRRLHRGAR

61 RRIERRKKRI KLLQELFSQE IAKTDEGFFQ RMKESPFYAE DKTILQENTL FNDKDFADKT

121 YHKAYPTINH LIKAWIENKV KPDPRLLYLA CHNIIKKRGH FLFEGDFDSE NQFDTSIQAL

181 FEYLREDMEV DIDADSQKVK EILKDSSLKN SEKQSRLNKI LGLKPSDKQK KAITNLISGN

241 KINFADLYDN PDLKDAEKNS ISFSKDDFDA LSDDLASILG DSFELLLKAK AVYNCSVLSK

301 VIGDEQYLSF AKVKIYEKHK TDLTKLKNVI KKHFPKDYKK VFGYNKNEKN NNNYSGYVGV

361 CKTKSKKLII NNSVNQEDFY KFLKTILSAK SEIKEVNDIL TEIETGTFLP KQISKSNAEI

421 PYQLRKMELE KILSNAEKHF SFLKQKDEKG LSHSEKIIML LTFKIPYYIG PINDNHKKFF

481 PDRCWVVKKE KSPSGKTTPW NFFDHIDKEK TAEAFITSRT NFCTYLVGES VLPKSSLLYS

541 EYTVLNEINN LQIIIDGKNI CDIKLKQKIY EDLFKKYKKI TQKQISTFIK HEGICNKTDE

601 VIILGIDKEC TSSLKSYIEL KNIFGKQVDE ISTKNMLEEI IRWATIYDEG EGKTILKTKI

661 KAEYGKYCSD EQIKKILNLK FSGWGRLSRK FLETVTSEMP GFSEPVNIIT AMRETQNNLM

721 ELLSSEFTFT ENIKKINSGF EDAEKQFSYD GLVKPLFLSP SVKKMLWQTL KLVKEISHIT

781 QAPPKKIFIE MAKGAELEPA RTKTRLKILQ DLYNNCKNDA DAFSSEIKDL SGKIENEDNL

841 RLRSDKLYLY YTQLGKCMYC GKPIEIGHVF DTSNYDIDHI YPQSKIKDDS ISNRVLVCSS

901 CNKNKEDKYP LKSEIQSKQR GFWNFLQRNN FISLEKLNRL TRATPISDDE TAKFIARQLV

961 ETRQATKVAA KVLEKMFPET KIVYSKAETV SMFRNKFDIV KCREINDFHH AHDAYLNIVV

1021 GNVYNTKFTN NPWNFIKEKR DNPKIADTYN YYKVFDYDVK RNNITAWEKG KTIITVKDML

1081 KRNTPIYTRQ AACKKGELFN QTIMKKGLGQ HPLKKEGPFS NISKYGGYNK VSAAYYTLIE

1141 YEEKGNKIRS LETIPLYLVK DIQKDQDVLK SYLTDLLGKK EFKILVPKIK INSLLKINGF

1201 PCHITGKTND SFLLRPAVQF CCSNNEVLYF KKIIRFSEIR SQREKIGKTI SPYEDLSFRS

1261 YIKENLWKKT KNDEIGEKEF YDLLQKKNLE IYDMLLTKHK DTIYKKRPNS ATIDILVKGK

1321 EKFKSLIIEN QFEVILEILK LFSATRNVSD LQHIGGSKYS GVAKIGNKIS SLDNCILIYQ

1381 SITGIFEKRI DLLKV

Fig. 2

a

b

c

1mm
10mm

d

1mm

e

3 ms
10-50 V
voltage (V)
3-11 repeats
time (ms)

Fig. 3

a
Relative fluorescence intensity
Survival rate (%)
Voltage (V)
Fluorescence intensity
Survival rate (%)
b
Relative fluorescence intensity
Survival rate (%)
repeats of pulses (times)
Fluorescence intensity
Survival rate

Fig. 4

expected efficiency of mRNA introduction
voltage application duration (ms)
20V
30V
40V
50V

Fig. 5

a

mCherry
Fluorescence intensity
40
30
20
10
0
x6
x+6-6
x alt±6
x7
x +3-3
x alt±3
N=16
N=12
N=10
N=14
N=10
N=9

b

Survival rate
100.0%
80.0%
60.0%
40.0%
20.0%
0.0%
x6
x +6-6
x alt+-6
x7
x +3-3
x alt+-3

c

x 6

x +3 -3

x alt±3

x +6 -6

x alt±6

x 12

Fig. 6

a
Fgf10
locus :
1237
aggagctcccaggagaggacaaaaacaaga AGG aaaaaca
1277
b
type I (no limb)
type II (limb defect)
type III (normal)
c
Cas9 (ng/µl)
gRNA (ng/µl)
400 200
200 100
100 50
50 25
34 4 1 n=39
28 3 7 n=38
13 6 22 n=41
1 2 21 n=24
0 20 40 60 80 100 (%)
type I
type II
type III

Fig. 7A

c
20 V, 2 pulses (interval: 100 ms)
50µs
200µs
1ms

a
Control
nucleus
mCherry
b
30 V, 2 pulses (interval: 100 ms)
50µs
100µs

Fig. 8

a
Fwd
Rev
Rosa26
locus
H2b
1
mCherry
711
76 tccgtgaacggccacgagttcgagatcgaGGGcgagggcg
115
ssODN: tccgtgaaTTCATAACTTCGTATAGCATACATTATACGAAGTTATcgagggcg
(117 bases)
EcoRI
loxP

b
Electroporation
Control
Bright field
mCherry

c
#1
#2
#3
#4
#5
#6
#7
#8
#9
#10
#11
# Control
WT, 497 bps
374 bps
138 bps
EcoRI digests

Fig. 9

a
Fwd2
Rev
Rosa26
locus
H2b
1
mCherry
711
76
tccgtgaacggccacgagttcgagatcgaGGGcgagggcg
115
ssODN:
(50 bases)
cgtgaacggccacgagttcgagatATcgagggcgagggcgagggccgccc
EcoRV

b
#1 #2 #3 #4 #5 #6
WT, 431 bp
341 bp
92 bp
EcoRV digests

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015031006 A
- US 8697359 B
- WO 2015049897 A


### Non-patent literature cited in the description


- **WANG, H. et al.** *Cell,* 2013, vol. 153, 910-918
- **GRABAREK, JB. et al.** *Genesis,* 2002, vol. 32, 269-276
- **SCARES, ML. et al.** *BMC Developmental Biology,* 2005, vol. 5, 28
- **KANEKO, T. et al.** *Scientific Reports,* 2014, vol. 4, 6382
- **PENG, H. et al.** *PLos ONE,* 2012, vol. 7 (8), e43748
- **OHNISHI Y. et al.** *Nucleic Acids Research,* 2010, vol. 38 (15), 5141-5151
- **MAZARI, E. et al.** *Development,* 2014, vol. 141, 2349-2359
- **YASUE A. et al.** *Scientific Reports,* 2014, vol. 4, 5705
- **YANG, H. et al.** *Cell,* 2013, vol. 154, 1370-1379
- **MASHIKO, D. et al.** *Scientific Reports,* 2013, vol. 3, 3355
- **GRABAREK, J. et al.** Efficient Delivery of dsRNA into Zona-Enclosed Mouse Oocytes and Preimplantation Embryos by Electroporation. *GENESIS,* 2002, vol. 32 (4), 269-276
- **KANEKO, T. et al.** Simple knockout by electroporation of engineered endonucleases into intact rat embryos. *Scientific Reports,* 2014, vol. 4 (6382), 1-5
- **PENG, H. et al.** Efficient Delivery of DNA and Morpholinos into Mouse Preimplantation Embryos by Electroporation. *PLOS ONE,* 2012, vol. 7 (8), 1-13
- **SAMUEL H. STERNBERG et al.** *Nature,* 2014, vol. 507, 62-67
- **YANG, H. et al.** *Cell,* 2013, vol. 154 (6), 1370-9
- Manipulating the Mouse Embryo: A Laboratory Manual. Cold Spring Harbor Press
- **SEKINE, K. et al.** *Nature Genetics,* 1999, vol. 21, 138-141
- **YASUE, A. et al.** *Scientific Reports,* 2014, vol. 4, 5705
- **ABE et al.** *Genesis,* 2011, vol. 49, 579-590